# EUROPEAN PATENT SPECIFICATION

(11) **EP 4 211 117 B1**
(45) Date of publication and mention of the grant of the patent: **02.12.2026**
(21) Application number: 21787124.3
(22) Date of filing: 09.09.2021
(51) Int. Cl.: C07D 231/38, C07C 235/60, C07C 255/44, C07F 5/02, C07F 5/04, C07C 255/30

(54) **PROCESSES AND INTERMEDIATES FOR THE PREPARATION OF (S)-5-AMINO-3-(4-((5-FLUORO-2-METHOXYBENZAMIDO)METHYL)PHENYL)-1-(1,1,1-TRIFLUOROPROPANE-2-YL)-1H-PYRAZOLE-4-CARBOXAMIDE**
VERFAHREN UND ZWISCHENPRODUKTE ZUR HERSTELLUNG VON (S)-5-AMINO-3-(4-(5-FLUOR-2-METHOXYBENZAMIDO)METHYL)PHENYL)-1-(1,1-TRIFLUORPROPAN-2-YL)-1H-PYRAZOL-4-CARBOXAMID
PROCÉDÉS ET INTERMÉDIAIRES POUR LA PRÉPARATION DE (S)-5-AMINO-3-(4-((5-FLUORO-2-MÉTHOXYBENZAMIDO)MÉTHYL)PHÉNYL)-1-(1,1,1-TRIFLUOROPROPANE-2-YL)-1 H-PYRAZOLE-4-CARBOXAMIDE

(30) Priority: 10.09.2020 US 202063076577 P
(43) Date of publication of application: 19.07.2023
(73) Proprietor: Loxo Oncology, Inc., Stamford, Connecticut 06901 (US)
(72) Inventor: ARGUELLES DELGADO, Alonso Jose, Indianapolis, Indiana 46206-6288 (US); EARY, Charles, Todd, Indianapolis, Indiana 46206-6288 (US); FENNELL, Jared, Wade, Indianapolis, Indiana 46206-6288 (US); FRANK, Scott, Alan, Indianapolis, Indiana 46206-6288 (US); MAGNUS, Nicholas, Andrew, Indianapolis, Indiana 46206-6288 (US); MCFADDIN, Elizabeth, Ann, Indianapolis, Indiana 46206-6288 (US); ROTHHAAR, Roger, Ryan, Indianapolis, Indiana 46206-6288 (US); SPENCER, Stacey, Renee, Indianapolis, Indiana 46206-6288 (US); VAID, Radhe Krishan, Indianapolis, Indiana 46206-6288 (US)
(74) Representative: Eli Lilly and Company Limited
(86) International application number: PCT/US2021/049621
(87) International publication number: WO 2022/056100

(56) References cited:
- EP-A1- 3 459 940
- WO-A1-2014/068527
- WO-A1-2017/103611
- CN-A- 107 652 294

## Description

### Background

The present invention relates to the fields of pharmaceutical chemistry and synthetic organic chemistry, and provides processes and key intermediates for the synthesis of (S)-5-amino-3-(4-((5-fluoro-2-methoxybenzamido)methyl)phenyl)-1-(1,1,1-trifluoro-propane-2-yl)-1H-pyrazole-4-carboxamide.

Bruton's Tyrosine Kinase (BTK) is a member of the src-related Tec family of cytoplasmic tyrosine kinases. BTK plays a key role in the B-cell antigen receptor signaling pathway, which is required for the development, activation and survival of normal white blood cells, known as B-cells. BTK also plays a critical role in the proliferation and survival of diverse B cell malignancies. Therefore, BTK is a molecular target useful for treatment across numerous B-cell leukemias and lymphomas including, for example, chronic lymphocytic leukemia, Waldenstrom macroglobulinemia, mantle cell lymphoma, and marginal zone lymphoma.

The compound, (S)-5-amino-3-(4-((5-fluoro-2-methoxybenzamido)methyl)phenyl)-1-(1,1,1-trifluoropropane-2-yl)-1H-pyrazole-4-carboxamide has the following structure and may be referred to herein as the compound of Formula (I):

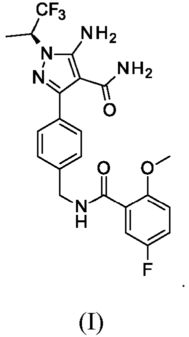

Hereinafter the compound of Formula (I) may also be referred to as (S)-5-amino-3-(4-((5-fluoro-2-methoxybenzamido)methyl)phenyl)-1-(1,1,1-trifluoropropane-2-yl)-1H-pyrazole-4-carboxamide; or 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide. The compound of Formula (I) is disclosed in WO 2017/103611 and/or WO 2020/028258. The compound of Formula (I) is a selective inhibitor of BTK. Formulations of the compound of Formula (I) are disclosed in WO 2020/028258.

The documents WO 2017/103611 and/or WO 2020/028258 noted above describe a synthesis method for the compound of Formula (I). The present disclosure provides a new process for preparing the compound of Formula (I). This new process provides an efficient, cost-effective, and facile synthesis of the compound of Formula (I), utilizing ecologically friendly reagents, allowing for optimal impurity control, and forming highly pure, crystalline materials. The pure, crystalline materials allow for facile purification of the product. Further, the present embodiments provide for novel intermediates that may be used to prepare the compound of Formula (I).

### Summary

The present embodiments provide for processes and new intermediates that may be used to prepare the compound of Formula (I).

One such embodiment includes a process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) comprising the steps of:
viii) coupling the compound of Formula (III): 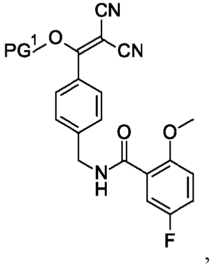 wherein PG¹ is -CH₃, -CH₂CH₃, -C(CH₃)₃, -CH₂CH=CH₂, methoxymethyl, tetrahydropyran, benzyl, trimethylsilyl, *tert*-butyl dimethylsilyl, di-*tert*-butylisobutylsilyl, di-*tert*-butyl[pyren-1-ylmethoxy]silyl, *tert*-butyl diphenylsilyl, acetyl, or benzoyl; and [(1S)-2,2,2-trifluoro-1-methyl-ethyl]hydrazine (8) thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
ix) synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof.

Another embodiment includes a process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) comprising the steps of:
viii) coupling the compound of Formula (III): 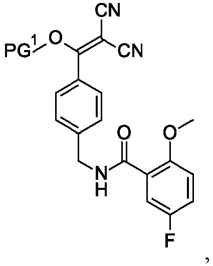 wherein PG¹ is -CH₃, -CH₂CH₃, -C(CH₃)₃, -CH₂CH=CH₂, methoxymethyl, tetrahydropyran, benzyl, trimethylsilyl, *tert*-butyl dimethylsilyl, di-*tert*-butylisobutylsilyl, di-*tert*-butyl[pyren-1-ylmethoxy]silyl, *tert*-butyl diphenylsilyl, acetyl, or benzoyl; and [(1S)-2,2,2-trifluoro-1-methyl-ethyl]hydrazine (8) thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof;
ix) synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methylethyl]pyrazol-3-yl]phenyl]methyl]-5-fluoro-2-methoxy-benzamide (10) or a salt thereof.; and
x) crystallizing (S)-5-amino-3-(4-((5-fluoro-2-methoxybenzamido)methyl)phenyl)-1-(1,1,1-trifluoropropane-2-yl)-1H-pyrazole-4-carboxamide (I) to provide a (S)-5-amino-3-(4-((5-fluoro-2-methoxybenzamido)methyl)phenyl)-1-(1,1,1-trifluoropropane-2-yl)-1H-pyrazole-4-carboxamide (I) in crystalline form.

Another embodiment is an intermediate referred to as the compound of Formula (II) and is shown below. The compound of Formula (II) is N-[[4-(2,2-dicyano-1-hydroxyvinyl)phenyl]methyl]-5-fluoro-2-methoxy-benzamide:

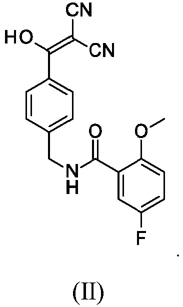

Accordingly, in another embodiment, the present process comprises employing the compound of Formula (II) to obtain the compound of Formula (I). In other words, described herein is a method of using N-[[4-(2,2-dicyano-1-hydroxy-vinyl)phenyl]methyl]-5-fluoro-2-methoxy-benzamide (II) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (III):

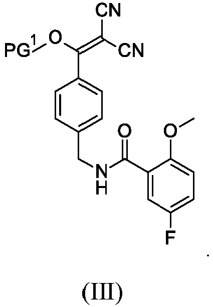

Wherein in Formula (III), "PG¹" refers to protecting group. Examples of what may constitute this PG¹ are -CH₃, -CH₂CH₃, -C(CH₃)₃, -CH₂CH=CH₂, methoxymethyl, tetrahydropyranyl, benzyl, silyl, acetyl, or benzoyl; or a pharmaceutically acceptable salt thereof. Silyl groups include but are not limited to trimethylsilyl, *tert*-butyl dimethylsilyl, di-*tert*-butylisobutylsilyl, di-*tert*-butyl[pyren-1-ylmethoxy]silyl, and *tert*-butyl diphenylsilyl.

A preferred embodiment of the present invention is made in which the compound of Formula (III) has the PG¹ being methyl. This compound is N-[[4-(2,2-dicyano-1-methoxy-vinyl)phenyl]methyl]-5-fluoro-2-methoxy-benzamide and is represented below as Formula (IIIA) :

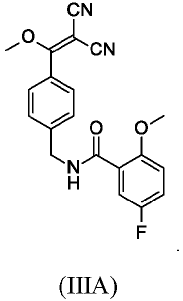

Accordingly, in one embodiment, the present process comprises employing the compound of Formula (III) to obtain the compound of Formula (I). In other words, the present embodiments include a method of using the compound of Formula (III) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I). In some embodiments, this may involve reacting the compound of Formula (IIIA) to obtain the compound of Formula (I).

The compound of Formula (II) may be prepared using the following Scheme I, which is described in greater detail herein:

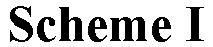

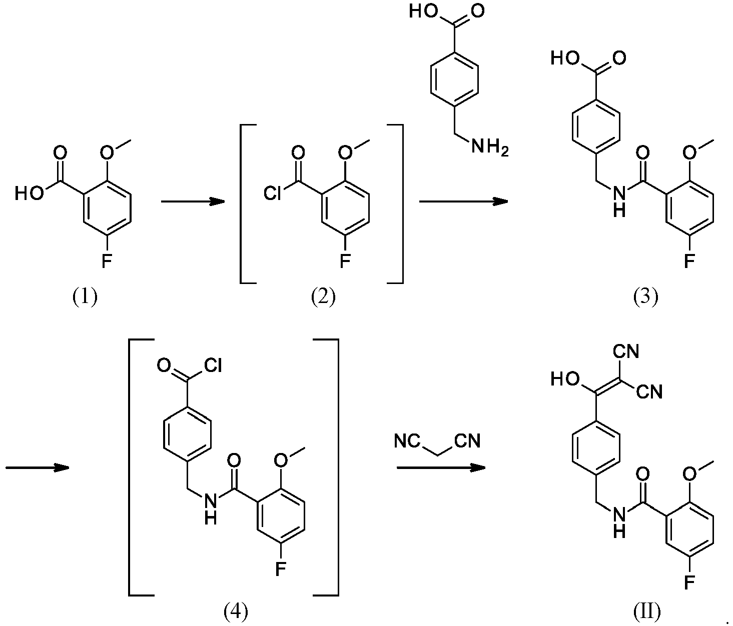

Additional embodiments include a more efficient and ecologically friendly method of producing the compound of Formula (I). Such embodiments may involve using the compound of Formula (II) and/or the compound of Formula (III).

Other embodiments may involve a process for the preparation of the compound of Formula (I), which involve using the reactions/compounds of Scheme II (which is described in greater detail herein). Scheme II uses the compound of Formula (II) and converts it into the compound of Formula (III), and then subsequently converts such compound into the compound of Formula (I):

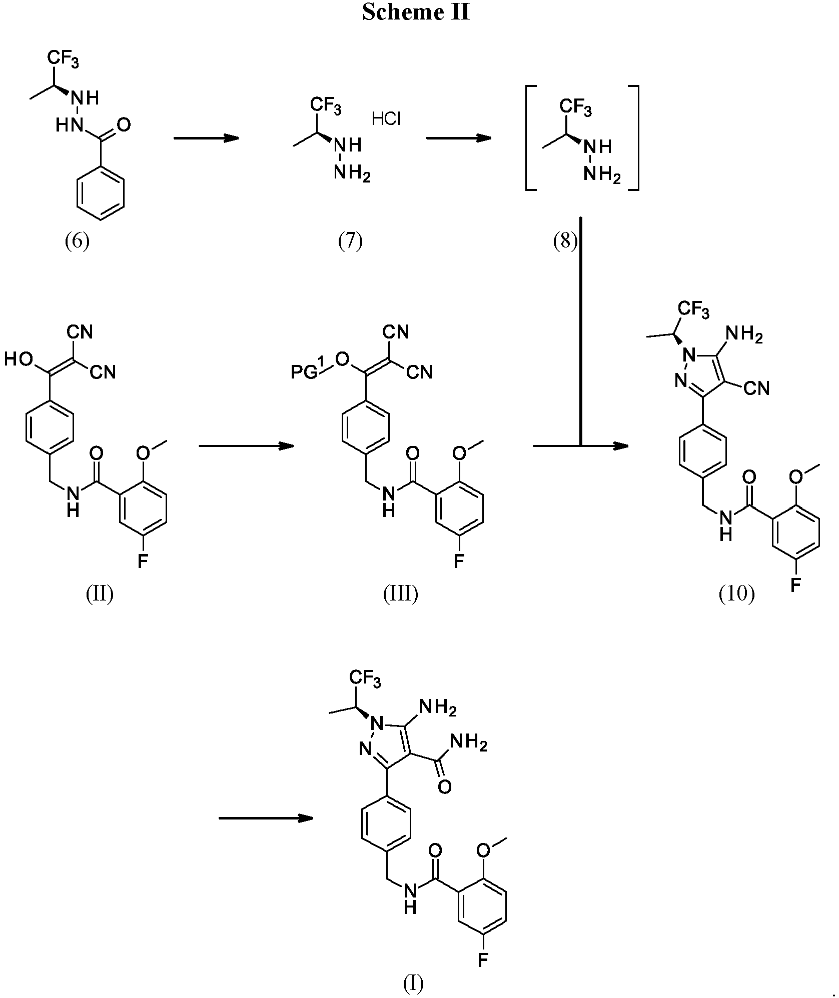

The embodiments shown in Scheme II are represented using the compound of Formula (III). As noted above, the compound of Formula (IIIA) is a sub-species of the compound of Formula (III), wherein the PG¹ is methyl. Those skilled in the art will appreciate that similar Scheme(s) may be used and constructed using other species as the PG¹ for the compound of Formula (III). All of these other embodiments (e.g., where a different PG¹ is used in Formula (III)) may be used to prepare the compound of Formula (I) using similar techniques and schemes as those disclosed herein.

As shown in Schemes I and II, process may include one or more of the following steps:
i) converting 5-fluoro-2-methoxy-benzoic acid (1) to give 5-fluoro-2-methoxybenzoyl chloride (2);
ii) coupling 5-fluoro-2-methoxy-benzoyl chloride (2) with 4-(aminomethyl)benzoic acid to give 4-[[(5-fluoro-2-methoxy-benzoyl)amino]methyl]benzoic acid (3) or a salt thereof;
iii) converting 4-[[(5-fluoro-2-methoxy-benzoyl)amino]methyl]benzoic acid (3) or a salt thereof to 4-[[(5-fluoro-2-methoxy-benzoyl)amino]methyl]benzoyl chloride (4);
iv) reacting 4-[[(5-fluoro-2-methoxy-benzoyl)amino]methyl]benzoyl chloride (4) with malononitrile to give N-[[4-(2,2-dicyano-1-hydroxy-vinyl)phenyl]methyl]-5-fluoro-2-methoxy-benzamide (II);
v) converting N'-[(1S)-2,2,2-trifluoro-1-methyl-ethyl]benzohydrazide (6) or a salt thereof to [(1S)-2,2,2-trifluoro-1-methyl-ethyl]hydrazine hydrochloride (7);
vi) converting [(1S)-2,2,2-trifluoro-1-methyl-ethyl]hydrazine hydrochloride (7) to [(1S)-2,2,2-trifluoro-1-methyl-ethyl]hydrazine (8);
vii) converting N-[[4-(2,2-dicyano-1-hydroxy-vinyl)phenyl]methyl]-5-fluoro-2-methoxy-benzamide (II) to the compound of Formula (III): 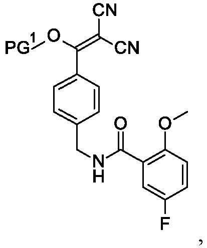 wherein PG¹ is -CH₃, -CH₂CH₃, -C(CH₃)₃, -CH₂CH=CH₂, methoxymethyl, tetrahydropyran, benzyl, trimethylsilyl, *tert*-butyl dimethylsilyl, di-*tert*-butylisobutylsilyl, di-*tert*-butyl[pyren-1-ylmethoxy]silyl, *tert*-butyl diphenylsilyl, acetyl, or benzoyl;
viii) reacting the compound of Formula (III): 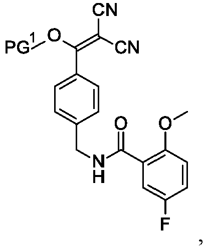 and [(1S)-2,2,2-trifluoro-1-methyl-ethyl]hydrazine (8) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof;
ix) synthesizing 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
x) optionally crystallizing 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide (I) to provide a 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide (I) in crystalline form.

Some embodiments of methods and processes whereby the above-recited compounds may be converted into the compound of Formula (I) will be described and shown herein.

### Description

Described herein is the compound, N-[[4-(2,2-dicyano-1-hydroxy-vinyl)phenyl]methyl]-5-fluoro-2-methoxy-benzamide:

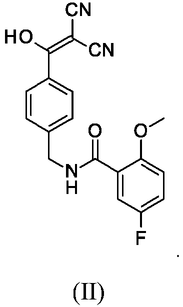

This compound of Formula (II) may be made according to the methods outlined herein. This compound of Formula (II) may be reacted to produce a compound of Formula (I). Specifically, after obtaining the compound of Formula (II), this compound of Formula (II) may be converted into the compound of Formula (I) using, for example the one or more of the following steps:
reacting the compound of Formula (II) to give N-[[4-(2,2-dicyano-1-methoxy-vinyl)phenyl]methyl]-5-fluoro-2-methoxy-benzamide (IIIA);
coupling N-[[4-(2,2-dicyano-1-methoxy-vinyl)phenyl]methyl]-5-fluoro-2-methoxy-benzamide (IIIA) and [(1S)-2,2,2-trifluoro-1-methyl-ethyl]hydrazine (8) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof;
synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
optionally crystallizing 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide (I) to provide (S)-5-amino-3-(4-((5-fluoro-2-methoxybenzamido)methyl)phenyl)-1-(1,1,1-trifluoropropane-2-yl)-1H-pyrazole-4-carboxamide (I) in crystalline form.

The reacting the compound of Formula (II) step above, involves the conversion of the compound of Formula (II) into the compound of Formula (III). In some embodiments, this may occur by reacting the compound of Formula (II) with a protecting group. Other ways of performing this reaction (which may be an alkylating reaction) may also be used.

The coupling N-[[4-(2,2-dicyano-1-methoxy-vinyl)phenyl]methyl]-5-fluoro-2-methoxy-benzamide (IIIA) and [(1S)-2,2,2-trifluoro-1-methyl-ethyl]hydrazine (8) step above may occur in basic conditions, although other conditions such as conversion directly from the hydrazine salt, may also be used.

Finally, as noted above, the compound of Formula (I) is obtained from the synthesizing step above. An optional crystallization step may be used to purify this compound. Of course, other ways and/or reactions and/or conditions may also be used to convert the compound of Formula (II) into the compound of Formula (I). Other purification methods, other than crystallization, may also be used.

Also described herein is the compound of Formula (III), which may be reacted and converted into the compound of Formula (I). In one embodiment, the compound of Formula (III) is the compound of Formula (IIIA), in which the PG¹ is methyl and is N-[[4-(2,2-dicyano-1-methoxy-vinyl)phenyl]methyl]-5-fluoro-2-methoxy-benzamide:

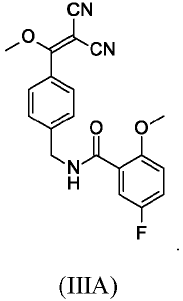

The compound of Formula (IIIA) may be converted into a compound of Formula (I). In one embodiment, this transformation occurs as follows:
coupling N-[[4-(2,2-dicyano-1-methoxy-vinyl)phenyl]methyl]-5-fluoro-2-methoxy-benzamide (IIIA) and [(1S)-2,2,2-trifluoro-1-methyl-ethyl]hydrazine (8) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof;
synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
optionally crystallizing (S)-5-amino-3-(4-((5-fluoro-2-methoxybenzamido)methyl)phenyl)-1-(1,1,1-trifluoropropane-2-yl)-1H-pyrazole-4-carboxamide (I) to provide a (S)-5-amino-3-(4-((5-fluoro-2-methoxybenzamido)methyl)phenyl)-1-(1,1,1-trifluoropropane-2-yl)-1H-pyrazole-4-carboxamide (I) in crystalline form.

As noted above, this coupling of N-[[4-(2,2-dicyano-1-methoxy-vinyl)phenyl]methyl]-5-fluoro-2-methoxy-benzamide (IIIA) and [(1S)-2,2,2-trifluoro-1-methyl-ethylhydrazine (8) step above may occur in basic conditions, although other conditions may also be used. Also, the compound of Formula (I) is obtained from the synthesizing step above. An optional crystallization step may be used to purify this compound. Of course, other ways and/or reactions and/or conditions may also be used to convert the compound of Formula (II) into the compound of Formula (I). Other purification methods, other than crystallization, may also be used.

The process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) described herein may be comprised of the steps below. For purposes of convenience, the compound numbers of Schemes I and II are included herein:
i) converting 5-fluoro-2-methoxy-benzoic acid (1) or a salt thereof to give 5-fluoro-2-methoxy-benzoyl chloride (2);
ii) coupling 5-fluoro-2-methoxy-benzoyl chloride (2) with 4-(aminomethyl)benzoic acid using a non-nucleophilic base to give 4-[[(5-fluoro-2-methoxy-benzoyl)amino]methyl]benzoic acid (3) or a salt thereof;
iii) converting 4-[[(5-fluoro-2-methoxy-benzoyl)amino]methyl]benzoic acid (3) or a salt thereof to 4-[[(5-fluoro-2-methoxy-benzoyl)amino]methyl]benzoyl chloride (4);
iv) reacting 4-[[(5-fluoro-2-methoxy-benzoyl)amino]methyl]benzoyl chloride (4) with malononitrile to give N-[[4-(2,2-dicyano-1-hydroxy-vinyl)phenyl]methyl]-5-fluoro-2-methoxy-benzamide (II);
v) deprotecting N'-[(1S)-2,2,2-trifluoro-1-methyl-ethyl]benzohydrazide (6) or a salt thereof to give [(1S)-2,2,2-trifluoro-1-methyl-ethyl]hydrazine hydrochloride (7);
vi) converting [(1S)-2,2,2-trifluoro-1-methyl-ethyl]hydrazine hydrochloride (7) under basic conditions to [(1S)-2,2,2-trifluoro-1-methyl-ethyl]hydrazine (8);
vii) converting N-[[4-(2,2-dicyano-1-hydroxy-vinyl)phenyl]methyl]-5-fluoro-2-methoxy-benzamide (II) with an alkylating reagent to give N-[[4-(2,2-dicyano-1-methoxy-vinyl)phenyl]methyl]-5-fluoro-2-methoxy-benzamide (IIIA);
viii) reacting N-[[4-(2,2-dicyano-1-methoxy-vinyl)phenyl]methyl]-5-fluoro-2-methoxy-benzamide (IIIA) and [(1S)-2,2,2-trifluoro-1-methyl-ethyl]hydrazine (8) under basic conditions to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof;
ix) synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
x) optionally crystallizing (S)-5-amino-3-(4-((5-fluoro-2-methoxybenzamido)methyl)phenyl)-1-(1,1, 1-trifluoropropane-2-yl)-1H-pyrazole-4-carboxamide (I) to provide a (S)-5-amino-3-(4-((5-fluoro-2-methoxybenzamido)methyl)phenyl)-1-(1,1, 1-trifluoropropane-2-yl)-1H-pyrazole-4-carboxamide (I) in crystalline form.

Step i) above involves converting 5-fluoro-2-methoxy-benzoic acid (1) or a salt thereof to 5-fluoro-2-methoxy-benzoyl chloride (2). In some embodiments, this reaction may be a chlorination (such as, for example, reaction with a chlorinating agent). Other conditions may also be used to effect this transformation. In some embodiments, converting 5-fluoro-2-methoxy-benzoic acid (1) or a salt thereof to 5-fluoro-2-methoxy-benzoyl chloride (2) may be accomplished under a variety of chlorination conditions. For example, thionyl chloride, oxalyl chloride, phosphorous(V) chloride, phosphorous(III) chloride, or other similar reagents may be employed. Those skilled in the art will appreciate that other reagents and/or conditions, such as transforming the carboxylic acid into an anhydride or activated ester group, may be used.

Step ii) above involves combining 5-fluoro-2-methoxy-benzoyl chloride (2) with 4-(aminomethyl)benzoic acid to give 4-[[(5-fluoro-2-methoxy-benzoyl)amino]methyl]benzoic acid (3) or a salt thereof. In some embodiments, this reaction may be an amide coupling reaction. Other conditions may also be used to effect this transformation. In some embodiments, combining 5-fluoro-2-methoxy-benzoyl chloride (2) with 4-(aminomethyl)benzoic acid to give 4-[[(5-fluoro-2-methoxy-benzoyl)amino]methyl]benzoic acid (3) or a salt thereof may be accomplished using a variety of non-nucleophilic bases. For example, triethylamine, diisopropylethylamine, or other similar reagents may be employed. Those skilled in the art will appreciate that other reagents and/or conditions may be used.

Step iii) above involves converting 4-[[(5-fluoro-2-methoxy-benzoyl)amino]methyl]benzoic acid (3) or a salt thereof to 4-[[(5-fluoro-2-methoxy-benzoyl)amino]methyl]benzoyl chloride (4). In some embodiments, this reaction may be a chlorination and may occur using a chlorinating agent. Other conditions may also be used to effect this transformation. In some embodiments, converting 4-[[(5-fluoro-2-methoxy-benzoyl)amino]methyl]benzoic acid (3) or a salt thereof with a chlorinating reagent to 4-[[(5-fluoro-2-methoxy-benzoyl)amino]methyl]benzoyl chloride (4) may be accomplished under a variety of chlorination conditions. For example, thionyl chloride, oxalyl chloride, phosphorous(V) chloride, phosphorous(III) chloride, or other similar reagents may be employed. Those skilled in the art will appreciate that other reagents and/or conditions, such as transforming the carboxylic acid into an anhydride or activated ester group, may be used.

Step iv) above involves combining 4-[[(5-fluoro-2-methoxy-benzoyl)amino]methyl]benzoyl chloride (4) with malononitrile to give N-[[4-(2,2-dicyano-1-hydroxy-vinyl)phenyl]methyl]-5-fluoro-2-methoxy-benzamide (II). In some embodiments, this reaction may be an amide coupling reaction and may be accomplished with a non-nucleophilic base. Other conditions may also be used to effect this transformation. In some embodiments, combining 4-[[(5-fluoro-2-methoxy-benzoyl)amino]methyl]benzoyl chloride (4) with malononitrile to give N-[[4-(2,2-dicyano-1-hydroxy-vinyl)phenyl]methyl]-5-fluoro-2-methoxy-benzamide (II) may be accomplished using a variety of non-nucleophilic bases. For example, triethylamine, diisopropylethylamine, or other similar reagents may be employed. Those skilled in the art will appreciate that other reagents and/or conditions may be used.

Step v) above involves reacting N'-[(1S)-2,2,2-trifluoro-1-methyl-ethyl]benzohydrazide (6) or a salt thereof to obtain [(1S)-2,2,2-trifluoro-1-methyl-ethyl]hydrazine hydrochloride (7). In some embodiments, this reaction may be a debenzoylation reaction. It may occur in either acidic or basic conditions. Other types of conditions may also be used to effect this transformation. In some embodiments, converting N'-[(1S)-2,2,2-trifluoro-1-methyl-ethyl]benzohydrazide (6) or a salt thereof to [(1S)-2,2,2-trifluoro-1-methylethyl]hydrazine hydrochloride (7) may be accomplished in acidic or basic conditions. For example, if acidic conditions are used, HCl or other similar reagents may be added. Alternatively, if basic conditions are used, reagents such as KOH, K₂CO₃, or other similar reagents may be added. Those skilled in the art will appreciate that other reagents and/or conditions may be used.

Step vi) above involves converting [(1S)-2,2,2-trifluoro-1-methyl-ethyl]hydrazine hydrochloride (7) to [(1S)-2,2,2-trifluoro-1-methyl-ethyl]hydrazine (8). In some embodiments, this reaction may be carried out under basic conditions. Other conditions may also be used to effect this transformation. In some embodiments, converting [(1S)-2,2,2-trifluoro-1-methyl-ethyl]hydrazine hydrochloride (7) to [(1S)-2,2,2-trifluoro-1-methylethyl]hydrazine (8) may be accomplished under a variety of basic conditions. For example, triethylamine, diisopropylethylamine, aqueous NaOH, aqueous LiOH, aqueous K₂CO₃, or other similar reagents may be employed. Those skilled in the art will appreciate that other reagents and/or conditions may be used.

Step vii) above involves converting N-[[4-(2,2-dicyano-1-hydroxy-vinyl)phenyl]methyl]-5-fluoro-2-methoxy-benzamide (II) to N-[[4-(2,2-dicyano-1-methoxy-vinyl)phenyl]methyl]-5-fluoro-2-methoxy-benzamide (IIIA). In some embodiments, this reaction may be an alkylation. Other conditions may also be used to effect this transformation. In some embodiments, N-[[4-(2,2-dicyano-1-hydroxy-vinyl)phenyl]methyl]-5-fluoro-2-methoxy-benzamide (II) to N-[[4-(2,2-dicyano-1-methoxy-vinyl)phenyl]methyl]-5-fluoro-2-methoxy-benzamide (IIIA) may be accomplished under a variety of alkylating conditions. For example, trimethyl orthoformate, methyl triflate, trimethylammonium tetrafluoroborate, N,N'-diisopropyl-O-methylisourea, or other similar reagents may be employed. Those skilled in the art will appreciate that other reagents and/or conditions may be used.

Step viii) above involves coupling N-[[4-(2,2-dicyano-1-methoxy-vinyl)phenyl]methyl]-5-fluoro-2-methoxy-benzamide (IIIA) and [(1S)-2,2,2-trifluoro-1-methylethyl]hydrazine (8) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10). In some embodiments, this reaction may be an annulation. Other conditions may also be used to effect this transformation. In some embodiments, coupling N-[[4-(2,2-dicyano-1-methoxy-vinyl)phenyl]methyl]-5-fluoro-2-methoxy-benzamide (IIIA) and [(1S)-2,2,2-trifluoro-1-methyl-ethyl]hydrazine (8) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof may be accomplished using a variety of non-nucleophilic bases. For example, triethylamine, diisopropylethylamine, or other similar reagents may be employed. Those skilled in the art will appreciate that other reagents and/or conditions may be used.

Step ix) above involves synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof. In some embodiments, this reaction may be a hydrolysis. Other conditions may also be used to effect this transformation. In some embodiments, synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof may be accomplished under acidic conditions using a variety of acids. For example, methanesulfonic acid, trifluoroacetic acid, hydrochloric acid, polyphosphoric acid, sulfuric acid, or other similar reagents may be employed. Hydrolysis may also be carried out under basic, oxidative, or metal catalyzed/stoichiometric conditions. For example, potassium tert-butoxide, sodium hydroxide, peroxides, ruthenium hydroxide, manganese dioxide, copper (II) acetate, Parkin's catalyst, MnO₂/SiO₂, or other similar reagents may be employed. Those skilled in the art will appreciate that other reagents and/or conditions such as enzymatic reactions or utilizing amidine intermediates, may be used.

The process for the preparation described herein may be further described wherein the chlorinating reagent of step i) is thionyl chloride, the non-nucleophilic base in step ii) is triethylamine, the chlorinating reagent in step iii) is thionyl chloride, the non-nucleophilic base of step iv) is triethylamine, the acid of step v) is hydrochloric acid and the temperature at which the reaction is carried out is 102 °C, the base of step vi) is triethylamine, the alkylating reagent of step vii) is trimethyl orthoformate and the temperature at which the reaction is carried out is 92 °C, the oxidative conditions of step ix) are aqueous methanesulfonic acid and the temperature at which the reaction is carried out is 85 °C, and the solvent of step x) is methanol. Preferred is a process for the preparation wherein the chlorinating agent in step i) is thionyl chloride. Preferred is a process for the preparation wherein the non-nucleophilic base in step ii) is triethylamine. Preferred is a process for the preparation wherein the chlorinating reagent in step iii) is thionyl chloride. Preferred is a process for the preparation wherein the non-nucleophilic base of step iv) is triethylamine. Preferred is a process for the preparation wherein the acid of step v) is hydrochloric acid and the temperature at which the reaction is carried out is 102 °C. Preferred is a process for the preparation wherein the base of step vi) is triethylamine. Preferred is a process for the preparation wherein the alkylating reagent of step vii) is trimethyl orthoformate and the temperature at which the reaction is carried out is 92 °C. Preferred is a process for the preparation wherein the oxidative conditions of step ix) are aqueous methanesulfonic acid and the temperature at which the reaction is carried out is 85 °C. Preferred is a process for the preparation wherein the solvent of step x) is methanol.

The following schemes (Schemes III - VI) detail synthetic routes which may be employed in the synthesis of the compound of Formula (I). Although the following routes have not been formally completed, it is believed that the following compounds could be made as follows:

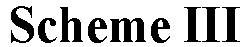

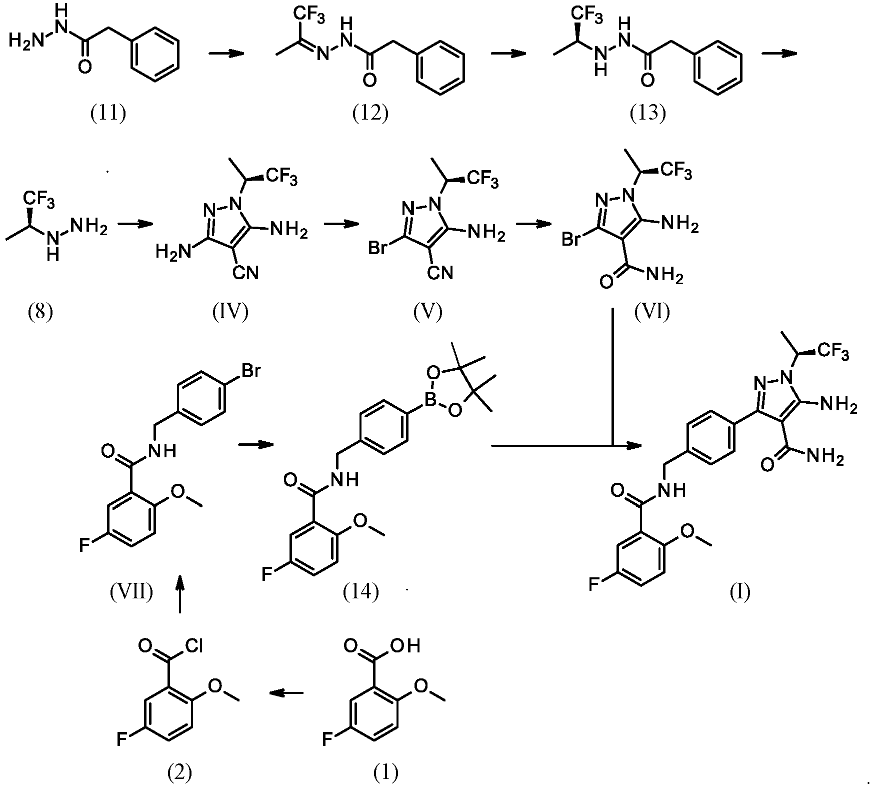

Hydrazide (11) or a salt thereof may be condensed with trifluoropropan-2-one in a polar aprotic solvent such as THF to give the hydrazone (12) or a salt thereof. Reduction of hydrazone (12) or a salt thereof may be effected by NaBH₄ or hydrogenation using a palladium or platinum catalyst to give hydrazide (13) or a salt thereof. Removal of the phenylacetate group may be achieved by heating under acidic conditions such as HCl in MeOH to give the hydrazine (8) which optionally may be isolated as the HCl salt. Hydrazine (8) or salt thereof may be reacted with potassium (dicyanoethenylidene)azanide by heating in a pressure vessel to give aminopyrazole (IV) or a salt thereof. A person of ordinary skill in the art will recognize that the annulation may be carried out directly from the hydrazine or a salt thereof. Conversion of the primary amine at the C-3 position of the pyrazole to the bromide may be achieved by using a variety of brominating agents, of which CuBr₂ may be used. Transformation of the nitrile moiety of pyrazole (V) or a salt thereof to carboxamide (VI) or a salt thereof may be achieved under mild conditions by use of a suitable hydride-platinum complex such as Ghaffar-Parkins catalyst or under basic conditions using H₂O₂, NaOH and polar solvents such as DMSO and EtOH. To obtain the precursor of the boronate ester (14), the amide coupling may be effected from either the acid chloride (2) under Schotten-Baumann conditions such as TEA in DCM or from benzoic acid (1) or a salt thereof directly using a suitable activating agent. A person of ordinary skill in the art would appreciate that activating agents include, but are not limited to, HATU, PyBOP, CDI, DCC, EDCI and T3P. The bromide moiety of amide (VII) may be converted to boronate ester (14) using a suitable catalyst such as palladium, rhodium or zinc in basic conditions and heating in a polar, aprotic solvent such as DMSO. Suzuki coupling of boronate ester (14) and bromide (VI) or a salt thereof using a palladium(0) source such as Pd(PPh₃)₄ or Pd₂(dba)₃ for example, and employing a base such as potassium or cesium carbonate may be used to give the compound of Formula (I).

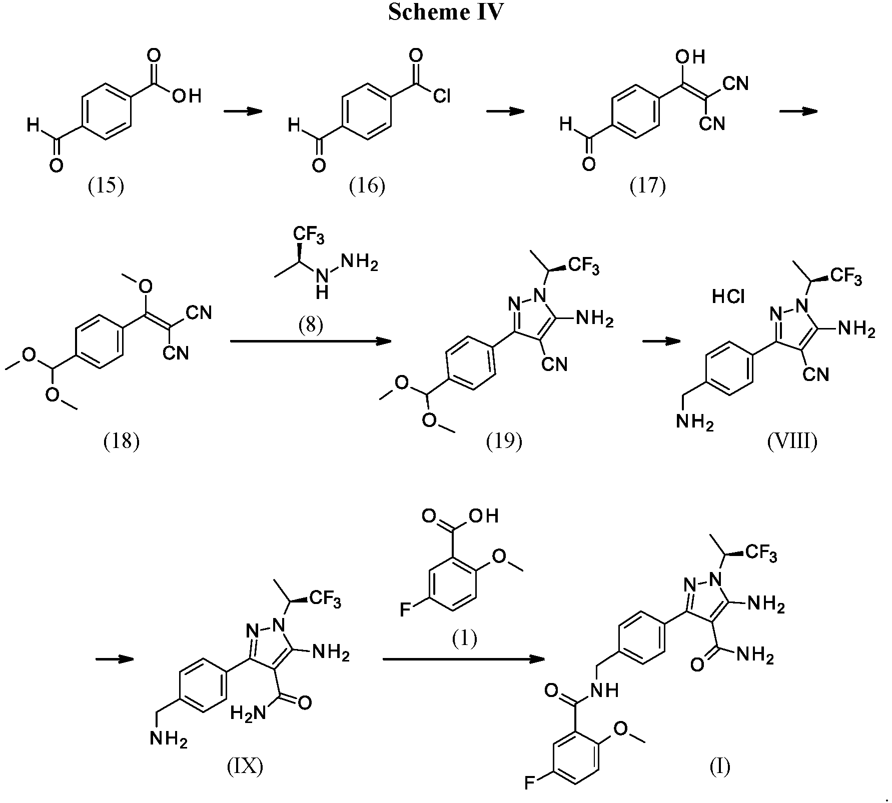

Benzoic acid (15) or a salt thereof may be converted to the corresponding acid chloride (16) using typical chlorinating conditions mentioned previously, among which, thionyl chloride, may be used. Reacting chloride (16) with malononitrile using NaH in a suitable solvent such as THF may be used that upon acidic work-up to give enol alcohol (17). A skilled artisan would recognize that alkylation of enol alcohol (17) may be effected with a mild base such as NaHCO₃ and a suitable alkylating agent, including previously mentioned trimethyl orthoformate or alternatively dimethylsulfate. Ring formation to substituted pyrazole (19) or a salt thereof may be carried out by addition by the aforementioned solution of hydrazine (8) or salt thereof to aryl enol ether (18). A skilled artisan will recognize that primary amine (VIII) may be synthesized from acetal (19) or a salt thereof via reductive amination following acidic hydrolysis. Previously mentioned hydrolysis conditions may be used to convert the nitrile group in substituted pyrazole (VIII) to give carboxamide (IX) or a salt thereof. Amide coupling of the amine moiety in (IX) or a salt thereof with benzoic acid (1) or a salt thereof may be utilized to give the compound of Formula (I).

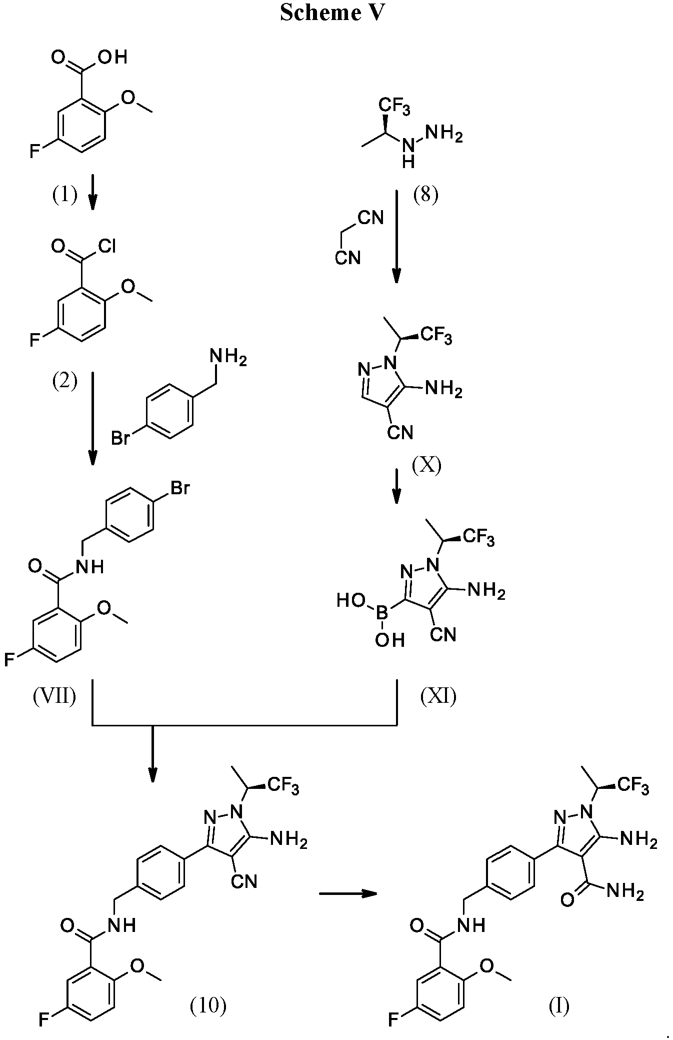

As previously mentioned, amide (VII) may be obtained from either acid chloride (2) using an amine base such as TEA or DIEA or from benzoic acid (1) or a salt thereof directly using a suitable activating agent also mentioned in the description for Scheme III. The annulation reaction of malononitrile and hydrazine (8) or a salt thereof using an amine base such as DIEA and heating in a protic solvent such as EtOH may afford pyrazole (X) or a salt thereof. Conversion to the boronic acid (XI) or a salt thereof or alternatively its ester, after installation of a suitable protecting group for the primary amine moiety such as a BOC group, may be effected by combining a bis-boronate source such as BISPIN, an iridium catalyst and a pyridine base in dioxane and heating to reflux to drive the reaction toward completion. Aryl coupling between bromide (VII) and boronic acid (XI) using previously mentioned Suzuki conditions in Scheme III may also be used to afford the compound of Formula (I).

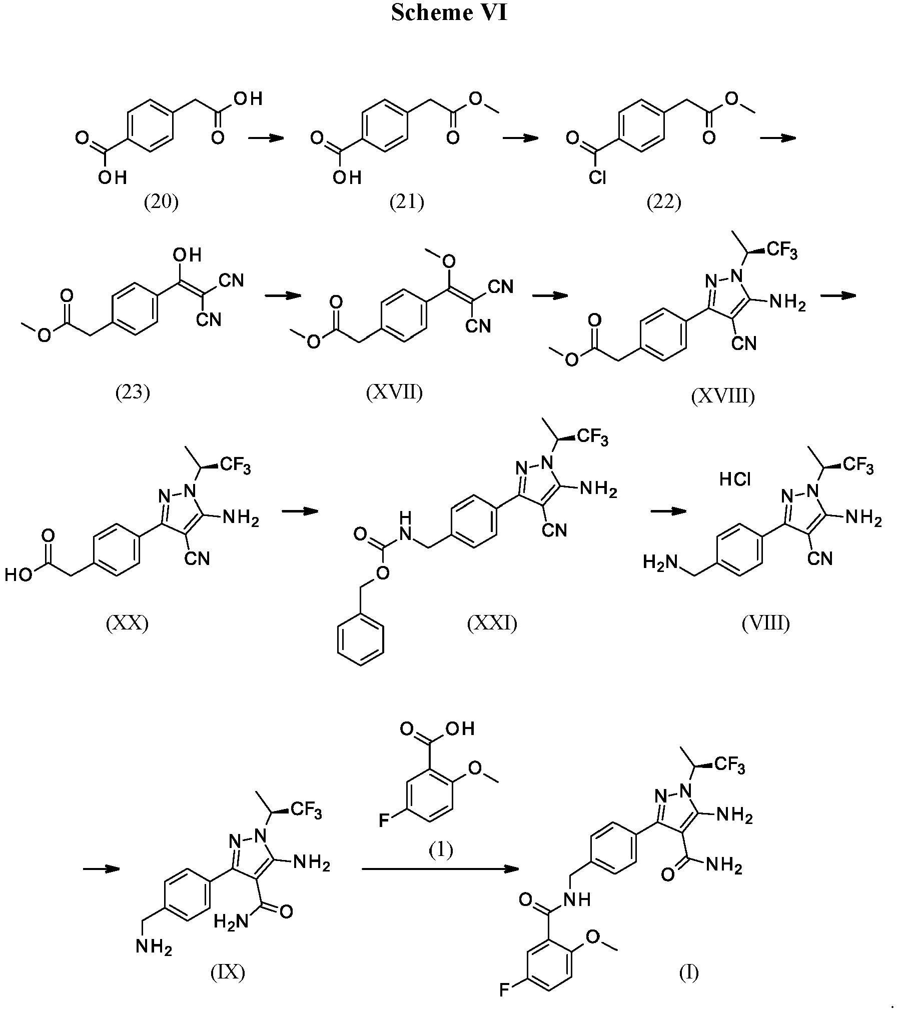

Ester (21) or a salt thereof may be obtained from carboxylic acid (20) or a salt thereof by using HCl gas dissolved in MeOH while maintaining a low temperature for both the reaction and subsequent work-up. Chlorination conditions mentioned in Scheme I using thionyl chloride or oxalyl chloride may afford chloride (22). Similarly, as in

Scheme IV, adding chloride (22) to a mixture of malononitrile and NaH in a suitable solvent such as THF may be used upon acidic work-up to give enol alcohol (23). Alkylation of enol (23) may be effected by using dimethylsulfate in refluxing THF to give enol ether (XVII). Annulation using hydrazine (8) or a salt thereof and an amine base such as TEA refluxing in a polar aprotic solvent such as THF may give pyrazole (XVIII) or a salt thereof. Selective hydrolysis of ester (XVIII) or a salt thereof using mild conditions of LiOH in aqueous MeOH may be used to give carboxylic acid (XX) or a salt thereof. Carbamate (XXI) or a salt thereof may be obtained by employing Curtius rearrangement conditions of DPPA, an appropriate alcohol, in this case benzyl alcohol, TEA and refluxing in toluene. Cleavage of the carbamate moiety may be effected by use of TMS-I in acetonitrile to give primary amine (VIII). Hydrolysis of the nitrile moiety of substituted pyrazole (VIII) under basic conditions using NaOH and H₂O₂ with a polar solvent combination such as DMSO and EtOH may afford carboxamide (IX) or a salt thereof. Amide coupling of amine (IX) or a salt thereof and benzoic acid (1) or a salt thereof may be used to give the compound of Formula (I).

The process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) described herein may be comprised of the steps below. For purposes of convenience, the compound numbers of Schemes III are included herein:
i) converting 2-phenylacetohydrazide (11) or a salt thereof to give 2-phenyl-N-[(Z)-(2,2,2-trifluoro-1-methyl-ethylidene)amino]acetamide (12) or a salt thereof;
ii) synthesizing 2-phenyl-N'-[(1S)-2,2,2-trifluoro-1-methyl-ethyl]acetohydrazide (13) from 2-phenyl-N-[(Z)-(2,2,2-trifluoro-1-methyl-ethylidene)amino]acetamide (12) or a salt thereof;
iii) converting 2-phenyl-N'-[(1S)-2,2,2-trifluoro-1-methyl-ethyl]acetohydrazide (13) or a salt thereof to [(1S)-2,2,2-trifluoro-1-methyl-ethyl]hydrazine (8);
iv) reacting [(1S)-2,2,2-trifluoro-1-methyl-ethyl hydrazine (8) or a salt thereof with dicyanoethenylideneazanide, or a pharmaceutically acceptable salt thereof, to give 3,5-diamino-1-[(1S)-2,2,2-trifluoro-1-methyl-ethyl]pyrazole-4-carbonitrile (IV) or a salt thereof;
v) converting 3,5 -diamino-1 -[(1 S)-2,2,2-trifluoro-1-methyl-ethyl]pyrazole-4-carbonitrile (IV) or a salt thereof to 5-amino-3-bromo-1-[(1S)-2,2,2-trifluoro-1-methyl-ethyl]pyrazole-4-carbonitrile (V) or a salt thereof;
vi) synthesizing 5-amino-3-bromo-1-[(1S)-2,2,2-trifluoro-1-methyl-ethyl]pyrazole-4-carboxamide (VI) or a salt thereof from 5-amino-3-bromo-1-[(1S)-2,2,2-trifluoro-1-methyl-ethyl]pyrazole-4-carbonitrile (V) or a salt thereof;
vii) converting 5-fluoro-2-methoxy-benzoic acid (1) or a salt thereof to give 5-fluoro-2-methoxy-benzoyl chloride (2);
viii) coupling 5-fluoro-2-methoxy-benzoyl chloride (2) with 4-bromo-benzylamine using a non-nucleophilic base to give N-[(4-bromophenyl)methyl]-5-fluoro-2-methoxy-benzamide (VII);
ix) synthesizing 5-fluoro-2-methoxy-N-[[4-(4,4,5,5-tetramethyl-1,3,2-dioxaborolan-2-yl)phenyl]methyl]benzamide (14) from N-[(4-bromophenyl)methyl]-5-fluoro-2-methoxy-benzamide (VII); and
x) coupling 5-fluoro-2-methoxy-N-[[4-(4,4,5,5-tetramethyl-1,3,2-dioxaborolan-2-yl)phenyl]methyl]benzamide (14) with 5-amino-3-bromo-1-[(1S)-2,2,2-trifluoro-1-methyl-ethyl]pyrazole-4-carboxamide (VI) or a salt thereof to give (S)-5-amino-3-(4-((5-fluoro-2-methoxybenzamido)methyl)phenyl)-1-(1,1,1-trifluoropropane-2-yl)-1H-pyrazole-4-carboxamide (I).

The process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) described herein may be comprised of the steps below. For purposes of convenience, the compound numbers of Schemes IV are included herein:
i) converting 4-formylbenzoic acid (15) or a salt thereof to give 4-formylbenzoyl chloride (16);
ii) coupling 4-formylbenzoyl chloride (16) with malononitrile under basic conditions to give 2-[(4-formylphenyl)-hydroxy-methylene]propanedinitrile (17);
iii) synthesizing 2-[[4-(dimethoxymethyl)phenyl]-methoxy-methylene]propanedinitrile (18) from 2-[(4-formylphenyl)-hydroxy-methylene]propanedinitrile (17);
iv) reacting 2-[[4-(dimethoxymethyl)phenyl]-methoxy-methylene]propanedinitrile (18) and [(1S)-2,2,2-trifluoro-1-methyl-ethyl]hydrazine (8) or a salt thereof to give 5-amino-3-[4-(dimethoxymethyl)phenyl]-1-[(1S)-2,2,2-trifluoro-1-methyl-ethyl]pyrazole-4-carbonitrile (19) or a salt thereof;
v) converting 5-amino-3-[4-(dimethoxymethyl)phenyl]-1-[(1S)-2,2,2-trifluoro-1-methyl-ethyl]pyrazole-4-carbonitrile (19) or a salt thereof to 5-amino-3-[4-(aminomethyl)phenyl]-1-[(1S)-2,2,2-trifluoro-1-methyl-ethyl]pyrazole-4-carbonitrile (VIII) or a salt thereof;
vi) synthesizing 5-amino-3-[4-(aminomethyl)phenyl]-1-[(1S)-2,2,2-trifluoro-1-methyl-ethyl]pyrazole-4-carboxamide (IX) from 5-amino-3-[4-(aminomethyl)phenyl]-1-[(1S)-2,2,2-trifluoro-1-methyl-ethyl]pyrazole-4-carbonitrile (VIII) or a salt thereof; and
vii) reacting 5-fluoro-2-methoxy-benzoic acid (1) or a salt thereof with 5-amino-3-[4-(aminomethyl)phenyl]-1-[(1S)-2,2,2-trifluoro-1-methyl-ethyl]pyrazole-4-carboxamide (IX) or a salt thereof to give (S)-5-amino-3-(4-((5-fluoro-2-methoxybenzamido)methyl)phenyl)-1-(1,1,1-trifluoropropane-2-yl)-1H-pyrazole-4-carboxamide (I).

The process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) described herein may be comprised of the steps below. For purposes of convenience, the compound numbers of Schemes V are included herein:
i) converting 5-fluoro-2-methoxy-benzoic acid (1) or a salt thereof to give 5-fluoro-2-methoxy-benzoyl chloride (2);
ii) coupling 5-fluoro-2-methoxy-benzoyl chloride (2) with 4-bromo-benzylamine using a non-nucleophilic base to give N-[(4-bromophenyl)methyl]-5-fluoro-2-methoxy-benzamide (VII);
iii) reacting [(1S)-2,2,2-trifluoro-1-methyl-ethyl hydrazine (8) or a salt thereof with malononitrile to give 5-amino-1-[(1S)-2,2,2-trifluoro-1-methylethyl]pyrazole-4-carbonitrile (X) or a salt thereof;
iv) converting 5-amino-1-[(1S)-2,2,2-trifluoro-1-methyl-ethyl]pyrazole-4-carbonitrile (X) or a salt thereof to give [5-amino-4-cyano-1-[(1S)-2,2,2-trifluoro-1-methyl-ethyl]pyrazol-3-yl]boronic acid (XI) or a salt thereof;
v) reacting N-[(4-bromophenyl)methyl]-5-fluoro-2-methoxy-benzamide (VII) with [5-amino-4-cyano-1-[(1S)-2,2,2-trifluoro-1-methyl-ethyl]pyrazol-3-yl]boronic acid (XI) or a salt thereof to give N-[[4-[5-amino-4-cyano-1-[(1S)-2,2,2-trifluoro-1-methyl-ethyl]pyrazol-3-yl]phenyl]methyl]-5-fluoro-2-methoxy-benzamide (10) or a salt thereof; and
vi) converting N-[[4-[5-amino-4-cyano-1-[(1S)-2,2,2-trifluoro-1-methylethyl]pyrazol-3-yl]phenyl]methyl]-5-fluoro-2-methoxy-benzamide (10) or a salt thereof to give (S)-5-amino-3-(4-((5-fluoro-2-methoxybenzamido)methyl)phenyl)-1-(1,1,1-trifluoropropane-2-yl)-1H-pyrazole-4-carboxamide (I).

The process for the preparation of (S)-5-amino-3-(4-((5-fluoro-2-methoxybenzamido)methyl)phenyl)-1-(1,1,1-trifluoropropane-2-yl)-1H-pyrazole-4-carboxamide (I) described herein may be comprised of the steps below. For purposes of convenience, the compound numbers of Schemes VI are included herein:
i) converting 4-(2-methoxy-2-oxo-ethyl)benzoic acid (21) to give methyl 2-(4-chlorocarbonylphenyl)acetate (22);
ii) synthesizing methyl 2-[4-(2,2-dicyano-1-hydroxy-vinyl)phenyl]acetate (23) from methyl 2-(4-chlorocarbonylphenyl)acetate (22);
iii) alkylating methyl 2-[4-(2,2-dicyano-1-hydroxy-vinyl)phenyl]acetate (23) to give methyl 2-[4-(2,2-dicyano-1-methoxy-vinyl)phenyl]acetate (XVII);
iv) reacting methyl 2-[4-(2,2-dicyano-1-methoxy-vinyl)phenyl]acetate (XVII) with [(1S)-2,2,2-trifluoro-1-methyl-ethyl]hydrazine (8) or a salt thereof to give methyl 2-[4-[5-amino-4-cyano-1-[(1S)-2,2,2-trifluoro-1-methylethyl]pyrazol-3-yl]phenyl]acetate (XVIII) or a salt thereof;
v) converting methyl 2-[4-[5-amino-4-cyano-1-[(1S)-2,2,2-trifluoro-1-methylethyl]pyrazol-3-yl]phenyl]acetate (XVIII) or a salt thereof to give 2-[4-[5-amino-4-cyano-1-[(1S)-2,2,2-trifluoro-1-methyl-ethyl]pyrazol-3-yl]phenyl]acetic acid (XX) or a salt thereof;
vi) synthesizing benzyl N-[[4-[5-amino-4-cyano-1-[(1S)-2,2,2-trifluoro-1-methyl-ethyl]pyrazol-3-yl]phenyl]methyl]carbamate (XXI) or a salt thereof from 2-[4-[5-amino-4-cyano-1-[(1S)-2,2,2-trifluoro-1-methyl-ethyl]pyrazol-3-yl]phenyl]acetic acid (XX) or a salt thereof;
vii) converting synthesizing benzyl N-[[4-[5-amino-4-cyano-1-[(1S)-2,2,2-trifluoro-1-methyl-ethyl]pyrazol-3-yl]phenyl]methyl]carbamate (XXI) or a salt thereof to give 5-amino-3-[4-(aminomethyl)phenyl]-1-[(1S)-2,2,2-trifluoro-1-methyl-ethyl]pyrazole-4-carbonitrile (VIII) or a salt thereof;
viii) synthesizing 5-amino-3-[4-(aminomethyl)phenyl]-1-[(1S)-2,2,2-trifluoro-1-methyl-ethyl]pyrazole-4-carboxamide (IX) or a salt thereof from 5-amino-3-[4-(aminomethyl)phenyl]-1-[(1S)-2,2,2-trifluoro-1-methyl-ethyl]pyrazole-4-carbonitrile (VIII) or a salt thereof; and
ix) reacting 5-fluoro-2-methoxy-benzoic acid (1) with 5-amino-3-[4-(aminomethyl)phenyl]-1-[(1S)-2,2,2-trifluoro-1-methyl-ethyl]pyrazole-4-carboxamide (IX) or a salt thereof to give (S)-5-amino-3-(4-((5-fluoro-2-methoxybenzamido)methyl)phenyl)-1-(1,1, 1-trifluoropropane-2-yl)-1H-pyrazole-4-carboxamide (I).

In another embodiment, a different intermediate may be used to prepare the compound of Formula (I). Specifically, this intermediate is a compound of Formula (IV):

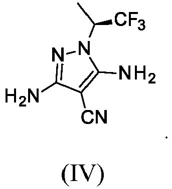

Accordingly, in one embodiment, the present process comprises employing the compound of Formula (IV) or a salt thereof to obtain the compound of Formula (I). In other words, described herein is a method of using 3,5-diamino-1-[(1S)-2,2,2-trifluoro-1-methyl-ethyl]pyrazole-4-carbonitrile (IV) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare a compound of Formula (I). Specifically, this intermediate is a compound of Formula (V): 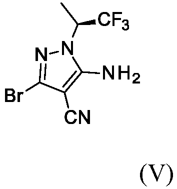 or a salt thereof.

Accordingly, in one embodiment, the present process comprises employing the compound of Formula (V) or a salt thereof to obtain the compound of Formula (I). In other words, described herein is a method of using 5-amino-3-bromo-1-[(1S)-2,2,2-trifluoro-1-methyl-ethyl]pyrazole-4-carbonitrile (V) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (VI): 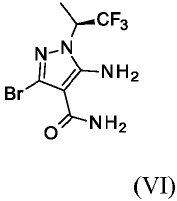 or a salt thereof.

Accordingly, in one embodiment, the present process comprises employing the compound of Formula (VI) or a salt thereof to obtain the compound of Formula (I). In other words, described herein is a method of using 5-amino-3-bromo-1-[(1S)-2,2,2-trifluoro-1-methyl-ethyl]pyrazole-4-carboxamide (VI) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare a compound of Formula (I). Specifically, this intermediate is a compound of Formula (VII):

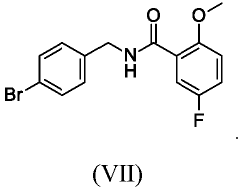

Accordingly, in one embodiment, the present process comprises employing the compound of Formula (VII) to obtain the compound of Formula (I). In other words, described herein is a method of using N-[(4-bromophenyl)methyl]-5-fluoro-2-methoxy-benzamide (VII) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare a compound of Formula (I). Specifically, this intermediate is a compound of Formula (VIII):

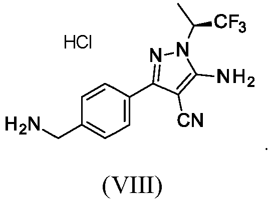

Accordingly, in one embodiment, the present process comprises employing the compound of Formula (VIII) to obtain the compound of Formula (I). In other words, described herein is a method of using 5-amino-3-[4-(aminomethyl)phenyl]-1-[(1S)-2,2,2-trifluoro-1-methyl-ethyl]pyrazole-4-carbonitrile hydrochloride (VIII) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (IX): 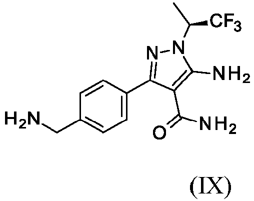 or a salt thereof.

Accordingly, in one embodiment, the present process comprises employing the compound of Formula (IX) or a salt thereof to obtain the compound of Formula (I). In other words, described herein is a method of using 5-amino-3-[4-(aminomethyl)phenyl]-1-[(1S)-2,2,2-trifluoro-1-methyl-ethyl]pyrazole-4-carboxamide (IX) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (X): 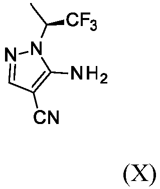 or a salt thereof.

Accordingly, in one embodiment, the present process comprises employing the compound of Formula (X) or a salt thereof to obtain the compound of Formula (I). In other words, described herein is a method of using 5-amino-1-[(1S)-2,2,2-trifluoro-1-methyl-ethyl]pyrazole-4-carbonitrile (X) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XI): 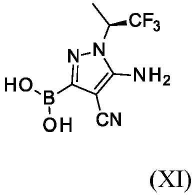 or a salt thereof.

Accordingly, in one embodiment, the present process comprises employing the compound of Formula (XI) or a salt thereof to obtain the compound of Formula (I). In other words, described herein is a method of using [5-amino-4-cyano-1-[(1S)-2,2,2-trifluoro-1-methyl-ethyl]pyrazol-3-yl]boronic acid (XI) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XII):

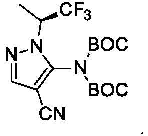

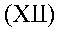

Accordingly, in one embodiment, the present process comprises employing the compound of Formula (XII) to obtain the compound of Formula (I). In other words, described herein is a method of using tert-butyl N-tert-butoxycarbonyl-N-[4-cyano-2-[(1S)-2,2,2-trifluoro-1-methyl-ethyl]pyrazol-3-yl]carbamate (XII) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XIII):

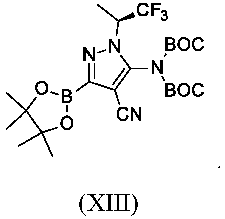

Accordingly, in one embodiment, the present process comprises employing the compound of Formula (XIII) to obtain the compound of Formula (I). In other words, described herein is a method of using *tert*-butyl N-*tert*-butoxycarbonyl-N-[4-cyano-5-(4,4,5,5-tetramethyl-1,3,2-dioxaborolan-2-yl)-2-[(1S)-2,2,2-trifluoro-1-methyl-ethyl]pyrazol-3-yl]carbamate (XIII) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare a compound of Formula (I). Specifically, this intermediate is a compound of Formula (XIV):

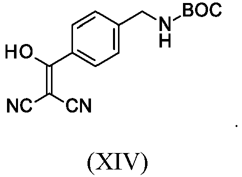

Accordingly, in one embodiment, the present process comprises employing the compound of Formula (XIV) to obtain the compound of Formula (I). In other words, described herein is a method of using *tert*-butyl N-[[4-(2,2-dicyano-1-hydroxy-vinyl)phenyl]methyl]carbamate (XIV) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XV):

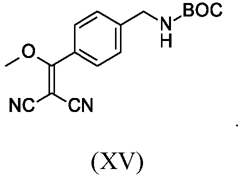

Accordingly, in one embodiment, the present process comprises employing the compound of Formula (XV) to obtain the compound of Formula (I). In other words, described herein is a method of using *tert*-butyl N-[[4-(2,2-dicyano-1-methoxy-vinyl)phenyl]methyl]carbamate (XV) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XVI): 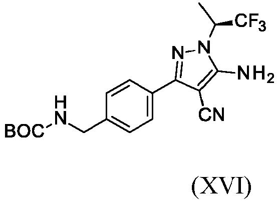 or a salt thereof.

Accordingly, in one embodiment, the present process comprises employing the compound of Formula (XVI) or a salt thereof to obtain the compound of Formula (I). In other words, described herein is a method of using *tert*-butyl N-[[4-[5-amino-4-cyano-1-[(1S)-2,2,2-trifluoro-1-methyl-ethyl]pyrazol-3-yl]phenyl]methyl]carbamate (XVI) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XVII):

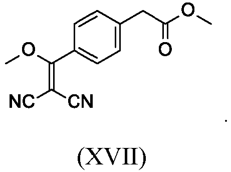

Accordingly, in one embodiment, the present process comprises employing the compound of Formula (XVII) to obtain the compound of Formula (I). In other words, described herein is a method of using methyl 2-[4-(2,2-dicyano-1-methoxy-vinyl)phenyl]acetate (XVII) in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XVIII): 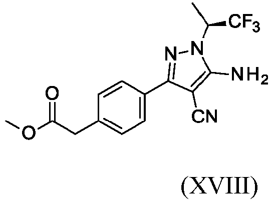 or a salt thereof.

Accordingly, in one embodiment, the present process comprises employing the compound of Formula (XVIII) or a salt thereof to obtain the compound of Formula (I). In other words, described herein is a method of using methyl 2-[4-[5-amino-4-cyano-1-[(1S)-2,2,2-trifluoro-1-methyl-ethyl]pyrazol-3-yl]phenyl]acetate (XVIII) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

In another embodiment, a different intermediate may be used to prepare the compound of Formula (I). Specifically, this intermediate is a compound of Formula (XIX): 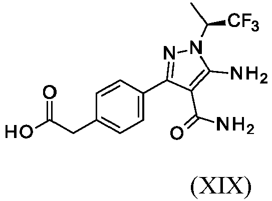 or a salt thereof.

Accordingly, in one embodiment, the present process comprises employing the compound of Formula (XIX) or a salt thereof to obtain the compound of Formula (I). In other words, described herein is a method of using 2-[4-[5-amino-4-carbamoyl-1-[(1S)-2,2,2-trifluoro-1-methyl-ethyl]pyrazol-3-yl]phenyl]acetic acid (XIX) or a salt thereof in the preparation of (S)-5-amino-3-(4-((5-fluoro-2-methoxybenzamido)methyl)phenyl)-1-(1,1,1-trifluoropropane-2-yl)-1H-pyrazole-4-carboxamide (I).

The reactions described herein may be performed via standard techniques known to the skilled artisan by employing routine glassware but also by using autoclave pressure chambers. These reactions also may be performed on pilot and/or production scale in equipment designed for such transformations. Further, each of these reactions described may be executed via either a batch process or flow reaction methodology. The term "batch process" as used herein refers to a process in which raw materials are combined in a reactor or vessel and product is removed at the end of the reaction. The term "continuous processing" or "flow reaction" as used herein refers to a process in which there is a continuous inflow of raw materials and outflow of product. Such continuous processing enables a platform where the final product may be synthesized by a fully continuous train of operations starting from initial starting materials.

Individual isomers, enantiomers, and diastereomers may be separated or resolved by one of ordinary skill in the art at any convenient point in the synthesis of compounds of Formula I by methods such as selective crystallization techniques or chiral chromatography (See for example, J. Jacques, et al., "Enantiomers, Racemates, and Resolutions", John Wiley and Sons, Inc., 1981, and E.L. Eliel and S.H. Wilen," Stereochemistry of Organic Compounds", Wiley-Interscience, 1994). Furthermore, tautomers may be found in certain compounds of the present invention. For example, compound (II) may exist in any ratio of the following isomeric forms: 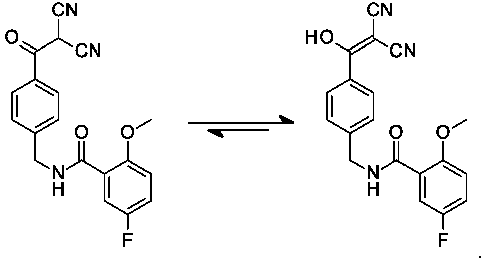 These forms are within the scope of the present embodiments.

Additionally, certain intermediates described in the following preparations may contain one or more nitrogen protecting groups. The variable protecting group may be the same or different in each occurrence depending on the particular reaction conditions and the particular transformations to be performed. The protection and deprotection conditions are well known to the skilled artisan and are described in the literature (See for example *"*Greene's Protective Groups in Organic Synthesis", Fourth Edition, by Peter G.M. Wuts and Theodora W. Greene, John Wiley and Sons, Inc. 2007). It is understood by the skilled artisan that compounds, intermediates, and pharmaceutically acceptable salts thereof described herein may equally be referred to by name, compound of Formula number, compound number, or the number from the Formula alone. E.g., Formula (III), or (III).

The compounds, or pharmaceutically acceptable salts thereof, prepared by the synthesis described herein may be prepared by a variety of procedures known in the art, some of which are illustrated in the Schemes, Preparations, and Examples below. For the avoidance of doubt, where the stereochemistry is not specified, all individual enantiomers, and mixtures thereof, as well as racemates are encompassed. The specific synthetic steps for each of the routes described may be combined in different ways, or in conjunction with steps from different schemes. The products of each step in the schemes below can be recovered by conventional methods well known in the art, including extraction, evaporation, precipitation, chromatography, filtration, trituration, and crystallization. The reagents and starting materials are readily available to one of ordinary skill in the art. Reactions are typically followed to completion using techniques known to the skilled artisan, for example TLC, HPLC, GC, LC/MS, RAMAN, and the like. The skilled artisan will appreciate that the technique used will depend on a variety of factors including the scale of the reaction, the type of vessel in which the reaction is performed, and the reaction itself.

The term "reacting" as used herein refers to the use of any suitable chemical reaction.

The abbreviations used herein are defined as follows: "DMSO" refers to dimethyl sulfoxide; "EtOAc" refers to ethyl acetate; "EtOH" refers to ethanol or ethyl alcohol; "GC" refers to gas chromatography; "HPLC" refers to high-performance liquid chromatography; "KF" refers to Karl Fischer assay; "LC/MS" refers to liquid chromatographymass spectrometry; "MeOH" refers to methanol or methyl alcohol; MsOH" refers to methanesulfonic acid; "MOM" refers to methoxymethyl ether; "RAMAN" refers to Raman spectroscopy; "RPM" refers to revolutions per minute; "TLC" refers to thin layer chromatography; "Tec" refers to tyrosine kinase expressed in hepatocellular carcinoma; and "THP" refers to tetrahydropyran; "DCM" refers to dichloromethane; "ACN" refers to acetonitrile; "Ghaffar-Parkins catalyst" refers to Hydrido(dimethylphosphinous acidkP)[hydrogen bis(dimethylphosphinito-kP)]platinum(II), CAS #173416-05-2; "DIEA" refers to diisopropylethylamine; "TEA" refers to triethylamine; "DMAP" refers to 4-dimethylaminopyridine; "TMS-I" refers to trimethylsilyl iodide; "DPPA" refers to diphenylphosphoryl azide; "FA" refers to formic acid; "BOC" refers to the tert-butyloxycarbonyl group; "BOC₂O" refers to Boc anhydride or tert-butoxycarbonyl tert-butyl carbonate; "rt" refers to room temperature; "BISPIN" refers to (E)-1-Pentene-1,2-diboronic acid bis(pinacol) ester, CAS #307531-75-5; "T3P" refers to 2,4,6-Tripropyl-1,3,5,2,4,6-trioxatriphosphorinane-2,4,6-trioxide; "PE" refers to petroleum ether or diethyl ether; "HATU" refers to N-[(dimethylamino)-1H-1,2,3-triazolo-[4,5-b]pyridin-1-ylmethylene]-N-methylmethanaminium hexafluorophosphate N-oxide, CAS #148893-10-1; "PyBOP" refers to (benzotriazol-1-yloxy)tripyrrolidinophosphonium hexafluorophosphate, CAS #128625-52-5; "TFA" refers to trifluoroacetic acid; "CDI" refers to 1,1'-carbonyldiimidazole; "DMF" refers to dimethylformamide; "DCC" refers to N, N'-dicyclohexylcarbodiimide; "EDCI" refers to 1-ethyl-3-(3-dimethylaminopropyl)carbodiimide; "dba" refers to dibenzylideneacetone group; "Fmoc" refers to fluorenylmethoxycarbonyl group; "Cbz" refers to carboxybenzyl group; "Bn" refers to benzyl group; "Tr" refers to trityl or triphenylmethyl group; and "Ts" refers to tosyl or toluenesulfonyl group.

The compound of Formula (I), (S)-5-amino-3-(4-((5-fluoro-2-methoxybenzamido)methyl)phenyl)-1-(1,1,1-trifluoropropane-2-yl)-1H-pyrazole-4-carboxamide, is prepared with N-[[4-(2,2-dicyano-1-methoxy-vinyl)phenyl]methyl]-5-fluoro-2-methoxy-benzamide (IIIA), as illustrated in Scheme II. The compound of Formula (II), N-[[4-(2,2-dicyano-1-hydroxy-vinyl)phenyl]methyl]-5-fluoro-2-methoxy-benzamide, is prepared beginning with 5-fluoro-2-methoxy-benzoic acid (1) or a salt thereof by the procedure illustrated in Scheme I.

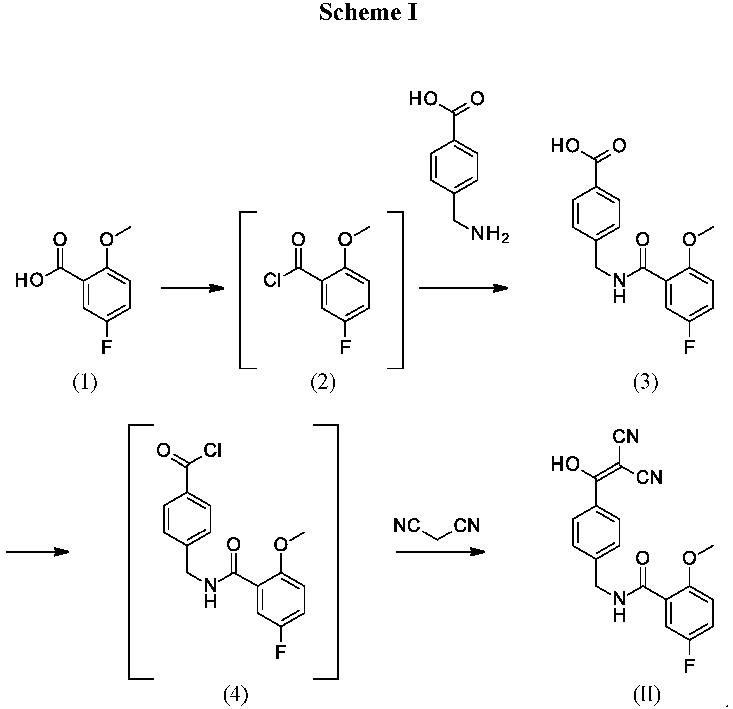

Substituted benzoic acid (1) or a salt thereof is dissolved in a suitable polar aprotic solvent and treated with an appropriate chlorinating reagent such as thionyl chloride, oxalyl chloride, or phosphorous pentachloride, to provide acyl chloride (2) as an un-isolated intermediate. 4-(Aminomethyl)benzoic acid is then coupled with acyl chloride (2) to furnish further substituted benzoic acid (3) or a salt thereof. Acyl chloride intermediate (4) may be synthesized under similar conditions to that of acyl chloride (2). Malononitrile, dissolved in an acceptable solvent and stirred until the mixture is homogeneous, is then added to aryl acyl chloride intermediate (4). This mixture is then added into a chilled solution of a non-nucleophilic base dissolved in an appropriate solvent over a period of time for sufficient conversion to aryl enol (II) or a salt thereof while maintaining a low reaction temperature. Aryl enol (II) or a salt thereof is then isolated by filtration after acidification of the reaction mixture creates an insoluble solid.

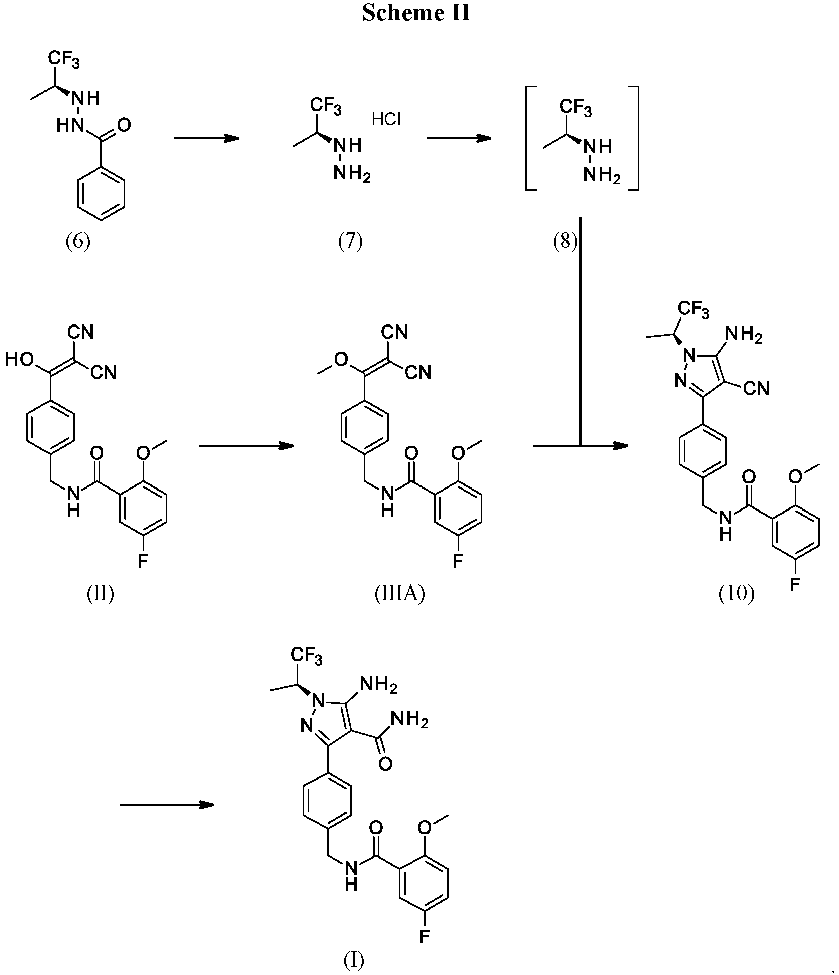

Aryl enol (II) is alkylated to aryl enol ether (III) using a suitable reagent such as trimethyl orthoformate and comparable reagents typically employed in the synthesis of enol ether moieties. Substituted hydrazine salt (7) is synthesized by reaction conditions previously disclosed in WO 17/103611. To a solution of (7), dissolved in an appropriate polar protic solvent and chilled, is added a non-nucleophilic base to form monosubstituted hydrazine (8). Annulation to substituted pyrazole (10) or a salt thereof is carried out by addition by the aforementioned solution of hydrazine (8) or a salt thereof to aryl enol ether (III) is similarly dissolved in a polar protic solvent and isolated by filtration. The nitrile of pyrazole (10) or a salt thereof is then hydrolyzed under aqueous, acidic conditions and heat to produce primary amide (I) which is isolated via filtration after pH of the reaction mixture is adjusted using an appropriate aqueous base. A skilled artisan may also recognize that this transformation may be carried out under basic conditions and/or in the presence of a metal catalyst. Crystallization and purification of (I) is accomplished through conditions previously disclosed in WO 2020/028258 to afford the compound of Formula (I) as a white, crystalline solid.

As noted above, the above-recited structure and scheme are given using Formula (IIIA). As noted above, Formula (IIIA) is a sub-species falling within the broader Formula (III). (In other words, in Formula (IIIA), the PG¹ is methyl). Those skilled in the art will appreciate that similar schemes and examples may be made using other species as the PG¹. The conversions that would then be used to remove the PG¹ and convert the compound into compound (10) or a salt thereof and/or ultimately into compound (I) are known to those skilled in the art.

The following schemes detail synthetic routes which may be employed in the synthesis of the compound of Formula (I). Although the following routes have not been formally completed, it is believed that the following compounds could be made as follows:

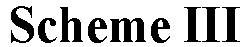

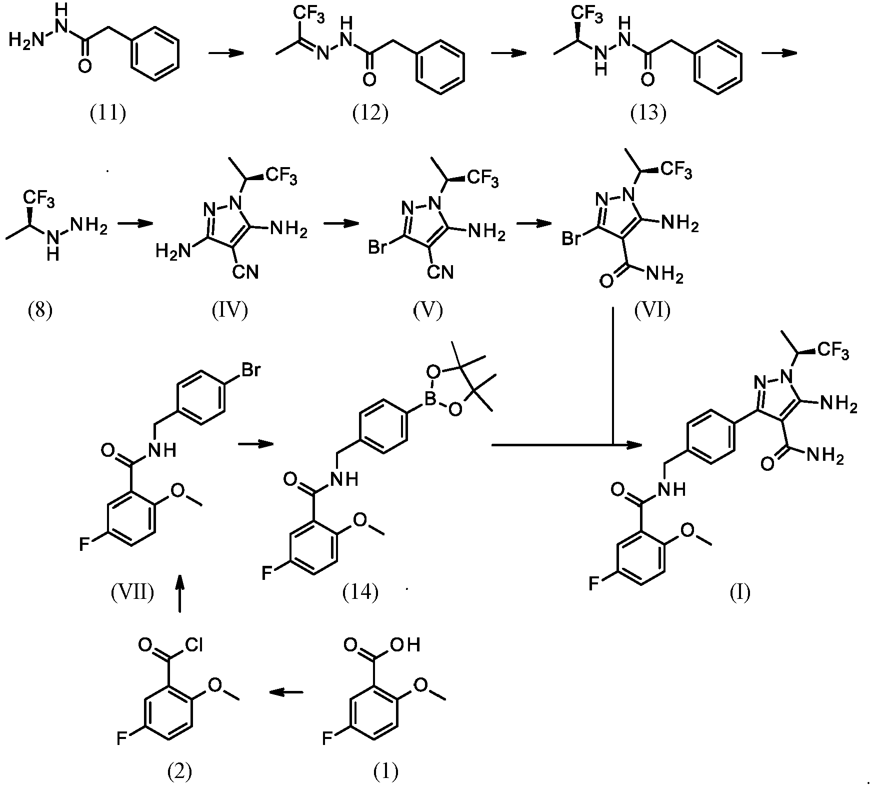

Hydrazide (11) or a salt thereof may be condensed with trifluoropropan-2-one in a polar aprotic solvent such as THF to give the hydrazone (12) or a salt thereof. Reduction of hydrazone (12) or a salt thereof may be effected by NaBH₄ or hydrogenation using a palladium or platinum catalyst to give hydrazide (13) or a salt thereof. Removal of the phenylacetate group may be achieved by heating under acidic conditions such as HCl in MeOH to give the hydrazine (8) which optionally may be isolated as the HCl salt. Hydrazine (8) or a salt thereof may be reacted with potassium (dicyanoethenylidene)azanide by heating in a pressure vessel to give aminopyrazole (IV) or a salt thereof. Conversion of the primary amine at the C-3 position of the pyrazole to the bromide may be achieved by using a variety of brominating agents, of which CuBr₂ may be used. Transformation of the nitrile moiety of pyrazole (V) or a salt thereof to carboxamide (VI) or a salt thereof may be achieved under mild conditions by use of a suitable hydride-platinum complex such as Ghaffar-Parkins catalyst or under basic conditions using H₂O₂, NaOH and polar solvents such as DMSO and EtOH. To obtain the precursor of the boronate ester (14), the amide coupling may be effected from either the acid chloride (2) under Schotten-Baumann conditions such as TEA in DCM or from benzoic acid (1) or a salt thereof directly using a suitable activating agent. A person of ordinary skill in the art would appreciate that activating agents include, but are not limited to, HATU, PyBOP, CDI, DCC, EDCI and T3P. The bromide moiety of amide (VII) may be converted to boronate ester (14) using a suitable catalyst such as palladium, rhodium or zinc in basic conditions and heating in a polar, aprotic solvent such as DMSO. Suzuki coupling of boronate ester (14) and bromide (VI) or a salt thereof using a palladium(0) source such as Pd(PPh₃)₄ or Pd₂(dba)₃ for example, and employing a base such as potassium or cesium carbonate may be used to give the compound of Formula (I).

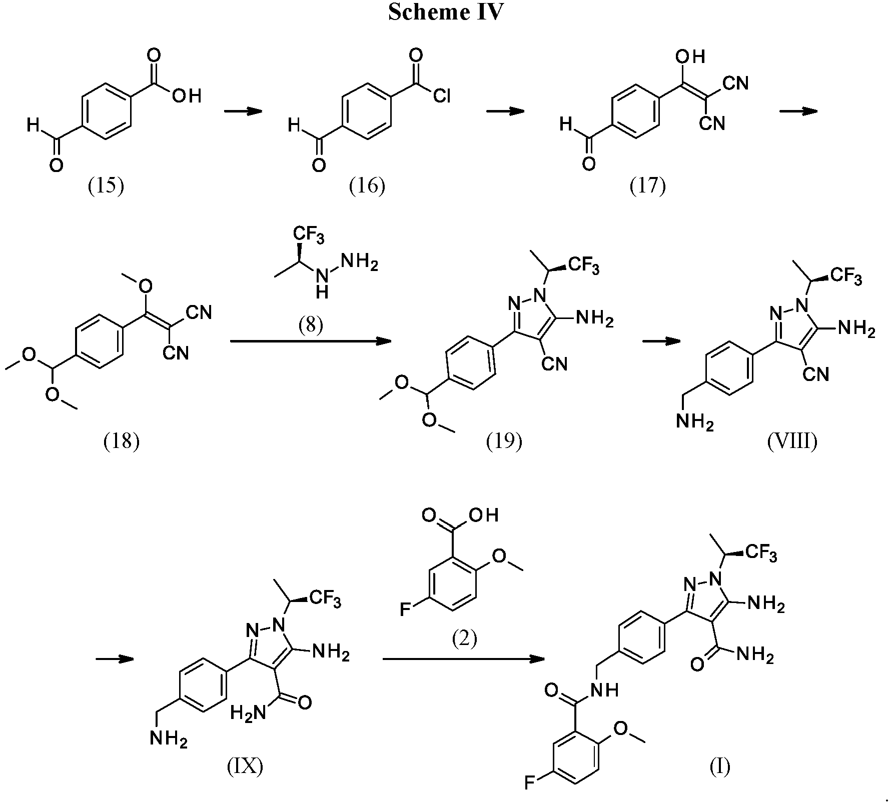

Benzoic acid (15) or a salt thereof may be converted to the corresponding acid chloride (16) using typical chlorinating conditions mentioned previously, among which, thionyl chloride, may be used. Reacting chloride (16) with malononitrile using NaH in a suitable solvent such as THF may be used that upon acidic work-up to give enol alcohol (17). A skilled artisan would recognize that alkylation of enol alcohol (17) may be effected with a mild base such as NaHCO₃ and a suitable alkylating agent, including previously mentioned trimethylorthoformate or alternatively dimethylsulfate. Ring formation to substituted pyrazole (19) or a salt thereof may be carried out by addition by the aforementioned solution of hydrazine (8) or a salt thereof to aryl enol ether (18). A skilled artisan will recognize that primary amine (VIII) may be synthesized from acetal (19) or a salt thereof via reductive amination following acidic hydrolysis. Previously mentioned hydrolysis conditions may be used to convert the nitrile group in substituted pyrazole (VIII) to give carboxamide (IX) or a salt thereof. Amide coupling of the amine moiety in (IX) or a salt thereof with benzoic acid (1) or a salt thereof may be utilized to give the compound of Formula (I).

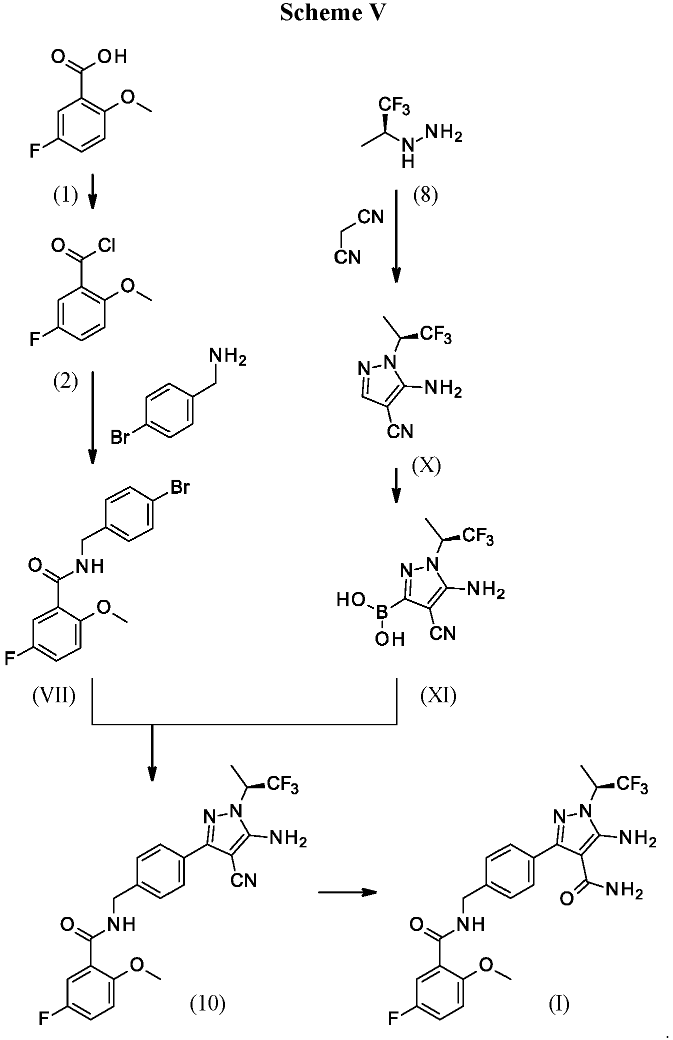

As previously mentioned, amide (VII) may be obtained from either acid chloride (2) using an amine base such as TEA or DIEA or from benzoic acid (1) or a salt thereof directly using a suitable activating agent also mentioned in the description for Scheme 3. The annulation reaction of malononitrile and hydrazine (8) or a salt thereof using an amine base such as DIEA and heating in a protic solvent such as EtOH may afford pyrazole (X) or a salt thereof. Conversion to the boronic acid (XI) or a salt thereof or alternatively its ester, after installation of a suitable protecting group for the primary amine moiety such as a BOC group, may be effected by combining a bis-boronate source such as BISPIN, an iridium catalyst and a pyridine base in dioxane and heating to reflux to drive the reaction toward completion. Aryl coupling between bromide (VII) and boronic acid (XI) or a salt thereof using previously mentioned Suzuki conditions in Scheme III may also be used to afford the compound of Formula (I).

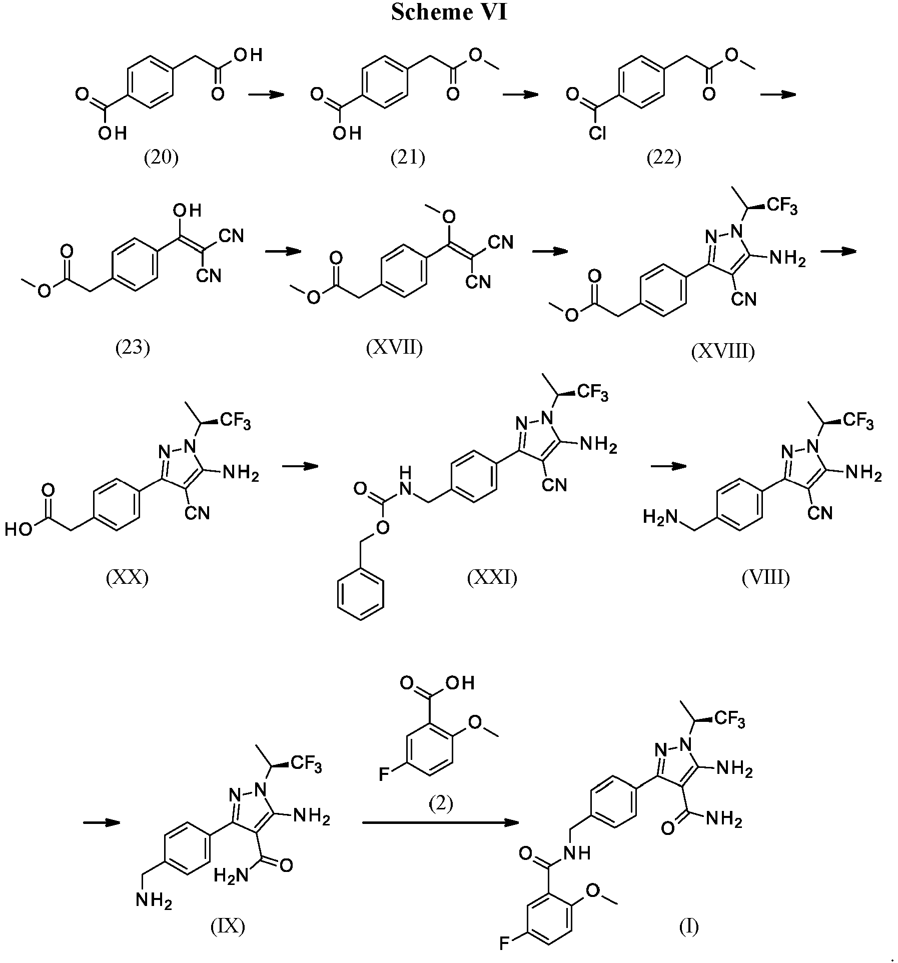

Ester (21) or a salt thereof may be obtained from carboxylic acid (20) or a salt thereof by using HCl gas dissolved in MeOH while maintaining a low temperature for both the reaction and subsequent work-up. Chlorination conditions mentioned in Scheme I using thionyl chloride or oxalyl chloride may afford chloride (22). Similarly, as in Scheme IV, adding chloride (22) to a mixture of malononitrile and NaH in a suitable solvent such as THF may be used upon acidic work-up to give enol alcohol (23). Alkylation of enol (23) may be effected by using dimethylsulfate in refluxing THF to give enol ether (XVII). Annulation using hydrazine (8) or a salt thereof and an amine base such as TEA refluxing in a polar aprotic solvent such as THF may give pyrazole (XVIII). Selective hydrolysis of ester (XVIII) or a salt thereof using mild conditions of LiOH in aqueous MeOH may be used to give carboxylic acid (XX) or a salt thereof. Carbamate (XXI) or a salt thereof may be obtained by employing Curtius rearrangement conditions of DPPA, an appropriate alcohol, in this case benzyl alcohol, TEA and refluxing in toluene. Cleavage of the carbamate moiety may be effected by use of TMS-I in acetonitrile to give primary amine (VIII). Hydrolysis of the nitrile moiety of substituted pyrazole (VIII) under basic conditions using NaOH and H₂O₂ with a polar solvent combination such as DMSO and EtOH may afford carboxamide (IX) or a salt thereof. Amide coupling of amine (IX) or a salt thereof and benzoic acid (1) or a salt thereof may be used to give the compound of Formula (I).

The following preparations and examples further illustrate the invention.

### Preparation 1

### [(1S)-2,2,2-trifluoro-1-methyl-ethyl]hydrazine hydrochloride

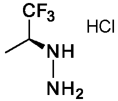

At rt N'-[(1S)-2,2,2-trifluoro-1-methyl-ethyl)]benzohydrazide (200 g, 8.61 mol), water (300 g, 166.53 mol), 35% conc. HCl (360 g, 34.50 mol, 35 w%) and m-xylene (150 mL) are added together. The contents are stirred and heated to 102 °C for 24 hours. The reaction is then cooled to 85 °C, toluene (1200 mL) is added, and the solution is gradually cooled to 25 °C. The layers are separated, and the organic layer discarded. The aqueous layer is washed with toluene (300 mL) and stirred at 25 °C for 30 minutes. The layers are separated, discarding the organic layer to give the title compound in the aqueous phase (709 g, 20 w%).

### Preparation 2

### N-[[4-(2,2-Dicyano-1-hydroxy-vinyl)phenyl]methyl]-5-fluoro-2-methoxy-benzamide

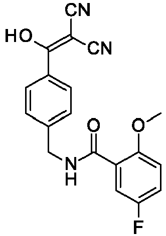

To a vessel 1, containing 4-[[(5-fluoro-2-methoxy-benzoyl)amino]methyl]benzoic acid (250 g, 824 mmol) at 25 °C under N₂ in ACN (2000 mL) is added dropwise thionyl chloride (117.7g, 989 mmol) and the mixture is stirred for 2 hours at 25 °C. The solution is concentrated to low volume and ACN (750 mL) is added and the solution is again concentrated to low volume. ACN (1000 mL) is added and the solution is stirred for 30 minutes while at 30 °C then ACN (250 mL) is added with malononitrile (81.7g, 1.24 mol). A solution of TEA (191.8 g, 1.90 mol) and ACN (250 mL) is added into an empty vessel 2, chilled to -5 °C and stirred for 120 minutes to achieve constant temperature. The acid chloride/malononitrile solution in vessel 1 is added into the triethylamine solution of vessel 2 while maintaining a temperature of -5 °C. After the addition is complete, the reaction is stirred for 15 hours at -10 °C. In a separate vessel, aqueous 1N HCl (1073 g, 1.285 HCl equivalents) is added and the temperature is adjusted to 10 °C then while maintaining the temperature at 10 °C this is added to the product solution in vessel 2 with continued stirring for 3 hours. The solids are filtered, and the filter cake washed with water. The solid wet cake (669.2g) is then split into two portions with one (535.4 g) wet cake to continue to the re-slurry in this experiment while the other wet cake portion (133.8 g) is dried and quality evaluated for research purposes. For the re-slurry, the first wet cake (535.4 g) is transferred into another vessel and ACN (700 mL) and water (1400 mL) is added. The mixture is heated to 40 °C and stirred for 15 hours. The temperature is lowered to 10 °C and stirred for 2 hours. The solids are filtered and washed with water. The solids are dried at 60-65 ° under vacuum to give the title compound (193.5 g, 551 mmol). ¹H NMR (400 MHz, DMSO-d₆) δ 3.89 (s, 3H), 4.52 (d, 2H), 7.18 (m, 1H), 7.20 (br, 1H), 7.34 (m, 1H), 7.36 (d, 2H), 7.51 (m, 1H), 7.57 (d, 2H), 8.85 (m, 1H).

### Preparation 3

### N-[[4-(2,2-Dicyano-1-methoxy-vinyl)phenyl]methyl]-5-fluoro-2-methoxy-benzamide

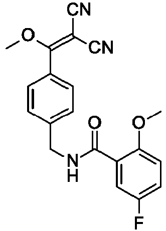

N-[[4-(2,2-Dicyano-1-hydroxy-vinyl)phenyl]methyl]-5-fluoro-2-methoxy-benzamide (300 g, 849 mmol) is added to trimethyl orthoformate (3 L, 270.0 mol). The mixture is stirred and heated to 92 °C for 18 hours. The solution is cooled to 40 °C then concentrated under vacuum to about 1200 g total solution while maintaining the temperature below 50 °C. The mixture is cooled to 20 °C to give the title compound (1200 g, 8.54 mmol, 26 wt% solution).

### Preparation 3a

### N-[[4-(2,2-Dicyano-1-methoxy-vinyl)phenyl]methyl]-5-fluoro-2-methoxy-benzamide

N-[[(4-(2,2-Dicyano-1-hydroxy-vinyl)phenyl]methyl]-5-fluoro-2-methoxy-benzamide (20 g, 56.9 mmol) and trimethyl orthoformate (190 g, 200 mL, 1790 mmol) are added together and the mixture is heated to 95 °C for 15 hours. The temperature is reduced to 40 °C and MeOH (200 mL) is added. Two hundred mL is distilled from the reaction mixture while maintaining 40 °C temperature using reduced pressure (200 mbar). The process of adding MeOH (200 mL) and distilling it off is repeated 6x giving an ending total solution volume of approximately 200 mL. The solution is seeded with N-[[4-(2,2-dicyano-1-methoxy-vinyl)phenyl]methyl]-5-fluoro-2-methoxy-benzamide, the temperature is allowed to cool to 22 °C, and the mixture is stirred overnight. When seeding with crystals as described herein, said crystals may be generated via a number of known techniques as would be appreciated by a skilled artisan. The resulting solids are collected by filtration and washed with MeOH (100 mL). The solids are dried at 50 °C under vacuum to give the title compound as an off-white solid (13.3 g, 36.4 mmol, 64% yield). ES/MS *m*/*z* 388 (M+Na), 366 (M+H), ¹H NMR 400 MHz, (DMSO-d₆) δ 3.89 (s, 3H), 3.90 (s, 3H), 4.60 (d, 2H), 7.19 (dd, 1H), 7.35 (m, 1H), 7.52 (dd, 1H), 7.55 (d, 2H), 7.65 (d, 2H), 8.93 (m, 1H).

### Preparation 4

### N-[[4-[(1S)-5-Amino-4-cyano-1-(2,2,2-trifluoro-1-methyl-ethyl)pyrazol-3-yl]phenyl]methyl]-5-fluoro-2-methoxy-benzamide

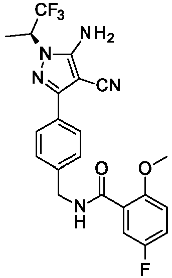

To N-[[4-(2,2-dicyano-1-methoxy-vinyl)phenyl]methyl]-5-fluoro-2-methoxy-benzamide (1200 g, 8.5 mol, 26 wt% solution) at 15 °C is charged 95% EtOH (1.14 L). In a separate vessel containing (1,1,1-trifluoropropan-2-yl)hydrazine hydrochloride (709 g total solution, 20 wt%) at 0 °C is added 95% EtOH (600 mL) followed by dropwise addition over 1 hour of TEA (390 g, 38.5 mol) while maintaining the temperature at 0-5 °C. The solution is recorded as pH=9. The (1,1,1-trifluoropropan-2-yl)hydrazine solution is added to N-[[4-(2,2-dicyano-1-methoxy-ethyl)phenyl]methyl]-5-fluoro-2-methoxy-benzamide solution dropwise over 1 hour while maintaining the temperature at 15-20 °C. The vessel containing (1,1,1-trifluoropropan-2-yl)hydrazine is rinsed into the reaction with 95% EtOH (510 mL) while at 15-20 °C. The mixture is stirred at 25 °C for 18 hours and water (1200 mL) is charged at 25 °C over 30 minutes. The solution is seeded with N-[[4-[(1S)-5-amino-4-cyano-1-(2,2,2-trifluoro-1-methyl-ethyl)pyrazol-3-yl]phenyl]methyl]-5-fluoro-2-methoxy-benzamide (1.5 g, 3.25 mmol) at 25 °C and stirred for 1 hour. Water (3120 mL) is charged at 25 °C over 3 hours and stirring is continued for an additional 3 hours. The solids are collected by filtration and washed with 28% EtOH in water (2x 1.4 L) and with water (1.5 L). 95% EtOH (3.0 L) is added to the collected wet cake, the mixture is heated to 65 °C, and stirred for 1 hour. The reaction is cooled to 55 °C and water is added (3.0 L) dropwise over 3 hours maintaining the temperature at 50-60 °C. The mixture is cooled to 21 °C and stirred at 21 °C for 60 hours. The solids are collected, washed with water (600 mL), and dried under vacuum at 55 °C for 24 hours to give the title compound as an off-white solid (336 g, 83% yield, 99.3% purity, 97.1% assay, 99.7% chiral purity). KF= 0.26 wt%, residual solvent EtOH 0.17 wt%, with non-detect for methyl formate, trimethyl orthoformate, toluene, MeOH, m-xylene. ¹H NMR (DMSO- d₆) δ 1.65 (d, 3H), 3.89 (s, 3H), 4.55 (d, 2H), 5.29 (m, 1H), 7.09 (s, 2H), 7.17 (dd, 1H), 7.33 (m, 1H), 7.43 (d, 2H), 7.51 (dd, 1H), 7.75 (d, 2H), 8.86 (m, 1H).

### Example 1

### 5-Amino-3-[4-[[(5-fluoro-2-methoxy-benzoyl)amino]methyl]phenyl]-1-[(1S)-2,2,2-trifluoro-1-methyl-ethyl]pyrazole-4-carboxamide

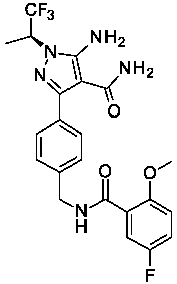

N-[[4-[5-Amino-4-cyano-1-[(1S)-2,2,2-trifluoro-1-methyl-ethyl]pyrazol-3-yl]phenyl]methyl]-5-fluoro-2-methoxybenzamide (20 g, 43.4 mmol), MsOH (80 mL, 1220 mmol), and water (1.50 g, 83.3 mmol) are added together and the mixture is heated with stirring to 85 °C. The reaction temperature is maintained at 85 °C for 6 hours, then cooled to 20 °C. In a separate vessel, water (100 mL) and NH₄OH in water (28 wt%, 200 mL, 1000 mmol) are charged and cooled to 0-10 °C. The acidic reaction mixture is slowly charged into the NH₄OH solution over 6-7 hours maintaining the temperature at 0-10 °C. The reaction is rinsed with MsOH (20 mL) for 30 minutes at 5-20 °C and added to the NH₄OH quench solution over 1-2 hours maintaining the temperature at 5-20 °C during the addition. The quenched reaction mixture is heated to 15-25 °C, EtOAc (140 mL) is charged, and the mixture is stirred at 15-25 °C for 30 minutes then allowed to stand for 30 minutes. The aqueous layer is removed. Water (100 mL) is added to the EtOAc solution at 20 °C with stirring for 30 minutes, then the layers are let stand for 30 minutes. The aqueous layer is separated. EtOAc (130 mL) is charged to the existing EtOAc solution and stirred at 20 °C for 30 minutes then the organic layer is concentrated to 140 mL under vacuum at temperatures under 50 °C. Additional EtOAc (120 mL), is charged, stirred at 20 °C for 30 minutes, then concentrated under vacuum to 140 mL total solution volume at a temperature under 50 °C. EtOH (120 mL) is charged and the mixture is concentrated to a 120 mL total solution volume at a temperature under 50 °C. The addition of EtOH (120 mL) and concentration to 120 mL total solution volume is repeated 2x. The solution temperature is adjusted to 42 °C and EtOH (12 mL) is charged and heated to 50 -60 °C. N-heptane (32 mL) is charged over 30 minutes at 50-60 °C. 5-Amino-3-[4-[[(5-fluoro-2-methoxy-benzoyl)amino]methyl]phenyl]-1-[(1S)-2,2,2-trifluoro-1-methyl-ethyl]pyrazole-4-carboxamide seed (0.40 g, 0.83 mmol) is charged and the mixture is stirred for 3-4 hours at 50-60 °C. A first portion of *n*-heptane (56 mL) is charged at 50-60 °C at a constant rate over 5 hours. A second portion of *n*-heptane (93 mL) is charged at 55 °C at a constant rate over 5 hours. The mixture is cooled to 15 °C for 4 hours and allowed to stir for an additional 4 hours. The solids are collected and the wet cake is dried at 50 °C for 66 hours to give title compound (17.5 g, 84% yield) as a white solid.

### Example 2

### 5-Amino-3-[4-[[(5-fluoro-2-methoxy-benzoyl)amino]methyl]phenyl]-1-[(1S)-2,2,2-trifluoro-1-methyl-ethyl]pyrazole-4-carboxamide

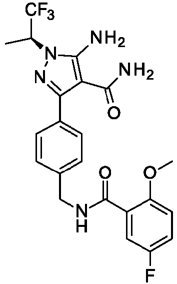

5-Amino-3-[4-[[(5-fluoro-2-methoxy-benzoyl)amino]methyl]phenyl]-1-[(1S)-2,2,2-trifluoro-1-methyl-ethyl]pyrazole-4-carboxamide (3.5 kg, 7.30 mol) is added to MeOH (17.5 L) and the solution is stirred and heated to 50-60 °C. The temperature is maintained at 50-60 °C for 1 hour and the solution polish filtered, rinsed with MeOH (3.5 L) and transferred to combine with the substrate solution. The temperature is adjusted to 55-65 °C and stirred for 0.5-1 hour. Water (9450 mL) is charged dropwise over 1-2 hours while maintaining the temperature at 55-65 °C. The temperature is adjusted to 50-60 °C with stirring at 91 RPM then 5-amino-3-[4-[[(5-fluoro-2-methoxy-benzoyl)amino]methyl]phenyl]-1-[(1S)-2,2,2-trifluoro-1-methyl-ethyl]pyrazole-4-carboxamide seed (35 g, 73 mmol) is added. Stirring is continued for 1-2 hours at 50-60 °C. Water (4.55 L) is charged dropwise over 8-10 hours while stirring at 50-60 °C. The mixture is then cooled to 5-15 °C for 5-7 hours and the temperature of the mixture is maintained at 5-15 °C for 2-4 hours. The solids are collected and washed with a MeOH: water (3:2) solution (2 x 3.5 L). The solids are dried for 6 hours under vacuum to give the title compound as an off-white solid (3312 g, 95% yield, 100% purity). ¹H NMR (400 MHz, DMSO-d₆) δ 1.62 (d, 3H), 3.89 (s, 3H), 4.56 (d, 2H), 5.30 (m, 1H), 6.68 (bs, 2H), 7.18 (dd, 1H), 7.33 (m, 1H), 7.43 (d, 2H), 7.47 (d, 2H), 7.52 (dd, 1H), 8.83 (m, 1H)

### Preparation 5

### 3,5-Diamino-1-[(1S)-2,2,2-trifluoro-1-methyl-ethyl]pyrazole-4-carbonitrile

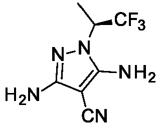

[(1S)-2,2,2-Trifluoro-1-methyl-ethyl]hydrazine hydrochloride (0.5 g, 3 mmol) and potassium (dicyanoethenylidene)azanide (0.4 g, 3 mmol) are combined in a pressure flask with water (2 mL) and heated to 100 °C overnight. The reaction is cooled to rt and a precipitate is formed. The precipitate is filtered, and the aqueous filtrate concentrated *in vacuo.* The residue is then dissolved in DCM (1 mL) and purified using silica gel chromatography (0-100% EtOAc in hexanes as the gradient eluent). Fractions containing product are combined and concentrated *in vacuo* to give the title compound (130 mg, 593 µmol, 20% yield). ES/MS *m*/*z* = 220.1 (M+H). ¹H NMR 400 MHz, (DMSO-d₆) δ 1.46 (d, J=1.00 Hz, 3H), 4.91 - 5.09 (m, 1H), 5.31 (s, 2H), 6.67 (s, 2H).

### Preparation 6

### 5-Amino-3-bromo-1-[(1S)-2,2,2-trifluoro-1-methyl-ethyl]pyrazole-4-carbonitrile

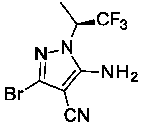

To 3,5-diamino-1-[(1S)-2,2,2-trifluoro-1-methyl-ethyl]pyrazole-4-carbonitrile (56.6 mg, 258 µmol) and ACN (2 mL) is added copper (II) bromide (57.7 mg, 12.1 µL, 258 µmol) and the mixture is stirred for 20 minutes cooling in a brine/ice bath. Then *tert-*butyl nitrite (26.6 mg, 30.8 µL, 258 µmol) is dissolved in ACN (2 mL) and is added dropwise to the reaction mixture. The reaction is stirred at -20 °C for 2 hours. Reaction is then diluted with water (6 mL) and the organics are extracted using EtOAc (3 x 20 mL) and dried over sodium sulfate, filtered, and are concentrated *in vacuo.* The residue is purified using silica gel chromatography (0-100% EtOAc in heptanes as the gradient eluent). Fractions containing product are concentrated *in vacuo* to give the title compound (21 mg, 74 µmol, 29% yield). ES/MS *m*/*z* (⁷⁹Br/⁸¹Br) = 283.00/285.00 (M+); ¹H NMR 400 MHz, (DMSO-d₆) δ 1.58 (d, J=1.00 Hz, 3H), 5.17 - 5.30 (m, 1H), 7.40 (s, 2H).

### Preparation 7

### 5-Amino-3-bromo-1-[(1S)-2,2,2-trifluoro-1-methyl-ethyl]pyrazole-4-carboxamide

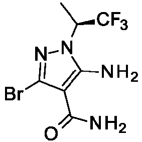

In a 20 mL reaction vial is combined 5-amino-3-bromo-1-[(1S)-2,2,2-trifluoro-1-methyl-ethyl]pyrazole-4-carbonitrile (16.5 mg, 58.3 µmol) and Ghaffar-Parkins catalyst (25.0 mg, 58.3 µmol) in EtOH (2 mL) and water (0.5 mL). The mixture is heated to 80 °C for 3 hours. After cooling to rt, the mixture is passed through a 0.45 µm filter and the solvent is removed under reduced pressure. The residue is purified using silica gel chromatography (0-10% MeOH with 0.1% NH₄OH in DCM as the gradient eluent). Product containing fractions are combined and concentrated *in vacuo* to give the title compound (12.5 mg, 41.5 µmol, 71% yield) as white solid. ES/MS *m*/*z* (⁷⁹Br/⁸¹Br) = 301.0/303.0 (M+); ¹H NMR 400 MHz, (DMSO-d₆) δ 1.56 (d, J=1.00 Hz, 3H), 5.18 - 5.39 (m, 1H), 6.54 (br s, 1H), 6.98 (s, 2H), 7.31 (br s, 1H).

### Preparation 8

### 5-Amino-1-[(1S)-2,2,2-trifluoro-1-methyl-ethyl]pyrazole-4-carbonitrile

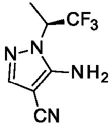

[(1S)-2,2,2-Trifluoro-1-methyl-ethyl]hydrazine hydrochloride (0.5 g, 3 mmol), DIEA (0.8 g, 1 mL, 6 mmol) and EtOH (25 mL) are combined in a round-bottom flask. The reaction mixture is stirred for 30 minutes until the hydrazine solids are dissolved. Then 2-(ethoxymethylene)propanedinitrile (0.4 g, 3 mmol) is added in portions to the reaction mixture and the reaction vessel is sealed. The reaction is stirred at 60 °C overnight. The reaction is concentrated *in vacuo* and purified using silica chromatography (0-100% EtOAc in hexanes as the gradient eluent). Fractions containing product are combined and concentrated *in vacuo* to give the title compound (385 mg, 1.89 mmol, 60% yield). ES/MS *m*/*z* = 204.9 (M+H); ¹H NMR 400 MHz, (DMSO-d₆) δ 1.58 (d, J=1.00 Hz, 3H), 5.13 - 5.30 (m, 1H), 7.00 (s, 2H), 7.66 (s, 1H).

### Preparation 9

### tert-Butyl N-tert-butoxycarbonyl-N-[4-cyano-2-[(1S)-2,2,2-trifluoro-1-methyl-ethyl]py-razol-3-yl]carbamate

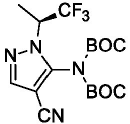

(S)-5-amino-1-(1,1,1-trifluoropropan-2-yl)-1H-pyrazole-4-carbonitrile (290 mg, 1 Eq, 1.42 mmol) is dissolved in THF (5 mL) in a round bottom flask. Then DMAP (17.4 mg, 0.1 Eq, 142 µmol), BOC₂O (620 mg, 653 µL, 2 Eq, 2.84 mmol), and TEA (431 mg, 594 µL, 3 Eq, 4.26 mmol) are added to the reaction. The reaction mixture is stirred at ambient temperature overnight. The reaction is quenched with sat. aq. NH₄Cl (15 mL) and is extracted with DCM (3 x 15 mL) through a phase separator frit. Organics are concentrated *in vacuo* and the residue is purified using silica chromatography (0-100% EtOAc in Hexanes as the gradient eluent). Product-containing fractions are combined and concentrated *in vacuo* to give the title compound (409.8 mg, 1.013 mmol, 71% yield). ¹H NMR 400 MHz, (DMSO-d₆) δ 7.82 (s, 1H), 4.58 (m, 1H), 1.68 - 1.66 (d, 3H), 1.41 (s, 9H), 1.37 (s, 9H).

### Preparation 10

### tert-Butyl N-tert-butoxycarbonyl-N-[4-cyano-5-(4,4,5,5-tetramethyl-1,3,2-dioxaborolan-2-yl)-2-[(1S)-2,2,2-trifluoro-1-methyl-ethyl]pyrazol-3-yl]carbamate

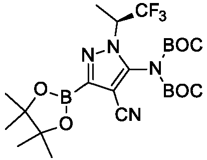

BISPIN (47 mg, 1.5 Eq, 0.19 mmol), tert-butyl N-*tert*-butoxycarbonyl-N-[4-cy-ano-2-[(1S)-2,2,2-trifluoro-1-methyl-ethyl]pyrazol-3-yl]carbamate (50 mg, 0.12 mmol), (1,5-Cyclooctadiene)(methoxy)iridium(I) dimer (1 mg, 2 µmol), and 4-tert-butyl-2-(4-tert-butyl-2-pyridyl)pyridine (1 mg, 4 µmol) are combined in a microwave vial with 1,4-dioxane (0.5 mL). The reaction vial is sealed and heated to 80 °C for 2 hours. The reaction is cooled to ambient temperature, diluted with DCM (20 mL) and then extracted with DCM (3 x 20 mL) through a phase separator frit. The organics are concentrated *in vacuo.* Then the residue is purified using silica chromatography (0-100% EtOAc in heptane as the gradient eluent). Product-containing fractions are combined and concentrated *in vacuo,* then dried under vacuum. The residue is suspended in pentane (4 mL), sonicated for 4 min, then the precipitate is isolated via filtration to give the title compound (20 mg, 38 µmol, 30% yield). ¹H NMR 400 MHz, (DMSO-d₆) δ 5.71 (m, 1H), 1.60 (d, 3H), 1.39 (s, 9H), 1.38 (s, 9H), 1.32 (S, 12H).

### Preparation 11

### N-[(4-bromophenyl)methyl]-5-fluoro-2-methoxy-benzamide

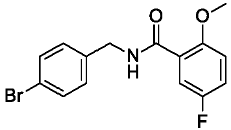

To a stirred mixture of 5-fluoro-2-methoxybenzoic acid (10.0 g, 58.8 mmol) and 4-bromo-benzylamine (10.9 g, 58.8 mmol) in DCM (150 mL) is added DIEA (22.8 g, 176.3 mmol) and T3P (44.9 g, 70.5 mmol, 50% in EtOAc) dropwise at rt under N₂. The resulting mixture is stirred for 1.5 hours at 50 °C under N₂. The mixture is allowed to cool down to rt. The reaction is quenched by the addition of water (150 mL) at rt. The resulting mixture is extracted with EtOAc (2 x 150 mL). The combined organic layers are washed with brine (2 x 100 mL) and dried over anhydrous Na₂SO₄. After filtration, the filtrate is concentrated under reduced pressure to give the title compound (17 g, 84% yield) as a yellow solid. ¹H NMR 300 MHz, (CDCl₃) δ 8.28 (s, 1H), 7.94 (dd, 1H), 7.51 - 7.41 (m, 2H), 7.31 - 7.20 (m, 2H), 7.18 - 7.11 (m, 1H), 6.93 (dd, 1H), 4.62 (d, 2H), 3.92 (s, 3H).

### Preparation 12

### tert-Butyl N-[[4-(2,2-dicyano-1-hydroxy-vinyl)phenyl]methyl]carbamate

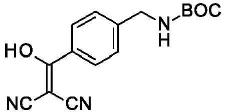

To a stirred mixture of 4-[(tert-butoxycarbonylamino)methyl]benzoic acid (10.0 g, 39.8 mmol) and malononitrile (3.39 g, 51.3 mmol) in DCM (200 mL) is added DIEA (25.7 g, 198.98 mmol) at rt under N₂. To the above mixture is added T3P (75.97 g, 119.4 mmol, 50% in EtOAc) dropwise over 30 minutes at rt. The resulting mixture is stirred for additional 2 hours at rt. The reaction is quenched with water (200 mL) and is extracted with DCM (3 x 200 mL). The combined organic layers are washed with sat. aq. NaCl (2 x 100 mL) and dried over anhydrous Na₂SO₄. After filtration, the filtrate is concentrated under reduced pressure. The residue is purified by silica gel column chromatography, eluting with DCM / MeOH (20:1-10:1) to give the title compound (10.5 g, 88%) as a dark-orange oil. ¹H NMR 400 MHz, (DMSO-d₆) δ 8.17 (s, 1H), 7.52 (d, 2H), 7.21 (d, 2H), 4.14 (d, 2H), 1.40 (s, 9H).

### Preparation 13

### tert-Butyl N-[[4-(2,2-dicyano-1-methoxy-vinyl)phenyl]methyl]carbamate

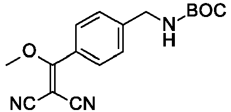

To a stirred solution of *tert*-butyl N-[[4-(2,2-dicyano-1-hydroxy-vinyl)phenyl]methyl]carbamate (10.5 g, 35.1 mmol) in ACN (150 mL) is added TEA (10.7 g, 105.2 mmol) in portions at rt under N₂. To the above mixture is added dimethyl sulfate (26.6 g, 210.5 mmol) in THF (2 mL) dropwise at rt. The resulting mixture is stirred for additional 3 hours at 50 °C. The mixture is allowed to cool down to rt. The reaction is quenched with water (200 mL) and extracted with EtOAc (2 x 200 mL). The combined organic layers are washed with brine (3 x 100 mL) and dried over anhydrous Na₂SO₄. After filtration, the filtrate is concentrated under reduced pressure. The residue is purified by silica gel column chromatography, eluting with PE/EtOAc (5:1-3:2) to give the title compound (10.9 g, 99% yield) as a dark-yellow oil. ¹H NMR 300 MHz, (DMSO-d₆) δ 7.67 - 7.59 (m, 2H), 7.46 (d, 2H), 4.24 (d, 2H), 3.89 (s, 3H), 1.41 (s, 9H).

### Preparation 14

### tert-Butyl N-[[4-[5-amino-4-cyano-1-[(1S)-2,2,2-trifluoro-1-methyl-ethyl]pyrazol-3-yl]phenyl]methyl]carbamate

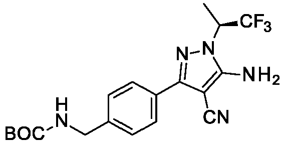

To a stirred solution of *tert*-butyl N-[[4-(2,2-dicyano-1-methoxy-vinyl)phenyl]methyl]carbamate (1.00 g, 3.191 mmol, 1.00 equiv) in THF (20 mL) is added [(1S)-2,2,2-trifluoro-1-methyl-ethyl]hydrazine hydrochloride (0.53 g, 3.2 mmol) and TEA (0.65 g, 6.38 mmol) at rt. The resulting mixture is stirred for 2 hours at 50 °C. The mixture is then allowed to cool down to rt. The resulting mixture is concentrated under reduced pressure. The residue is purified by silica gel column chromatography, eluting with PE/EtOAc (5:1-3:1) to give the title compound (1.2 g, 92% yield) as a yellow solid. ¹H NMR 400 MHz, (DMSO-d₆) δ 7.72 (d, 2H), 7.33 (d, 2H), 7.09 (s, 2H), 5.32 - 5.25 (m, 1H), 4.15 (d, 2H), 1.65 (d, 3H), 1.40 (s, 9H).

### Preparation 15

### 5-Amino-3-[4-(aminomethyl)phenyl]-1-[(1S)-2,2,2-trifluoro-1-methyl-ethyl]pyrazole-4-carbonitrile hydrochloride

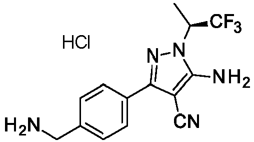

Into a 25 mL round-bottom flask is added *tert*-butyl N-[[4-[5-amino-4-cyano-1-[(1S)-2,2,2-trifluoro-1-methyl-ethyl]pyrazol-3-yl]phenyl]methyl]carbamate (1.20 g, 2.93 mmol) and HCl (4M in 1,4-dioxane, 7 mL) at rt. The resulting mixture is stirred for 1 hour at rt. The mixture is concentrated under vacuum and then is washed with Et₂O (3 x 5 mL) and again is concentrated under vacuum to give the crude title compound. The crude product is used in the next step directly without further purification. ES/MS *m*/*z* = 310.1 [M+H]⁺. ¹H NMR 400 MHz, (DMSO-d₆) δ 8.50 (s, 2H), 7.84 - 7.71 (m, 2H), 7.64 - 7.53 (m, 2H), 7.20 (s, 2H), 5.45 - 5.38(m, 1H), 4.08 - 4.04 (m, 2H), 1.65 (d, 3H).

### Preparation 16

### 5-Amino-3-[4-(aminomethyl)phenyl]-1-[(1S)-2,2,2-trifluoro-1-methyl-ethyl]pyrazole-4-carboxamide

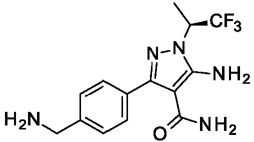

To a stirred mixture of 5-amino-3-[4-(aminomethyl)phenyl]-1-[(1S)-2,2,2-tri-fluoro-1-methyl-ethyl]pyrazole-4-carbonitrile (120 mg, 0.388 mmol) and NaOH (77.6 mg, 1.94 mmol) in DMSO (1 mL) and EtOH (6 mL) is added H₂O₂ (0.7 ml, 30% in H₂O) dropwise at rt. The resulting mixture is then stirred for 2 hours at 50 °C. The mixture is allowed to cool down to rt and then is concentrated under vacuum. The crude product (100 mg) was purified by Prep-HPLC (XBridge Prep C18 OBD^{™} Column, 19 ×150 mm, 5µm; Mobile Phase A: Water (10 mmol/L NH₄HCO₃), Mobile Phase B: ACN; Flow rate: 25 mL/min; Gradient: 10% B to 26% B in 6 min, 26% B; Wavelength: 254/220 nm). The product containing fraction is lyophilized to give the title compound (15.2 mg, 12% yield) as a white solid. ES/MS *m*/*z* = 328.2 [M+H]⁺. ¹H NMR 400 MHz, (DMSO-d₆) δ 7.55 - 7.31 (m, 4H), 5.21 (q, 1H), 4.19 (t, 0.5H), 3.78 (t, 1.5H), 1.75-1.50 (m, 3H).

### Preparation 17

### [5-Amino-4-cyano-1-[(1S)-2,2,2-trifluoro-1-methyl-ethyl]pyrazol-3-yl]boronic acid

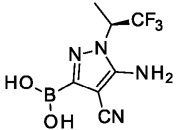

*tert*-Butyl N-*tert*-butoxycarbonyl-N-[4-cyano-5-(4,4,5,5-tetramethyl-1,3,2-diox-aborolan-2-yl)-2-[(1S)-2,2,2-trifluoro-1-methyl-ethyl]pyrazol-3-yl]carbamate (25 mg, 47 µmol) is dissolved in DCM (1 mL) and is treated with TFA (0.54 g, 0.36 mL, 4.7 mmol). The reaction is stirred at ambient temperature for 3 hours. The product is purified directly without workup using silica chromatography (0-100% EtOAc in hexanes as the gradient eluent). Product-containing fractions are combined and concentrated *in vacuo* to give the title compound (7 mg, 0.03 mmol, 60% yield). ¹H NMR 400 MHz, (DMSO-d₆) δ .67 (d, J=1.00 Hz, 3H), 5.33 - 5.58 (m, 1H), 9.03 (br s, 2H), 11.56 (s, 1H) 12.46 (s, 1H).

### Preparation 18

### 4-(2-Methoxy-2-oxo-ethyl)benzoic acid

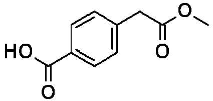

To a stirred solution of HCl (gas) in MeOH (1000 mL, 0.3 N) is added 4-(carboxymethyl)benzoic acid (50 g, 278 mmol) at 0 °C. The mixture is stirred for 1 hour at 0 °C. The resulting mixture is concentrated under reduced pressure keeping the temperature below 20 °C to give a residue. The residue is re-crystallized from PE/EtOAc (120 mL/40 mL) to give the title compound (40.0 g, 74% yield) as an off-white solid. ¹H NMR 400 MHz, (DMSO-d₆) δ 12.93 (s, 1H), 7.91 (d, 2H), 7.40 (d, 2H), 3.79 (s, 2H), 3.63 (s, 3H).

### Preparation 19

### Methyl 2-[4-(2,2-dicyano-1-methoxy-vinyl)phenyl]acetate

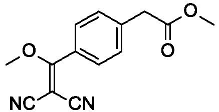

To a stirred solution of 4-(2-methoxy-2-oxo-ethyl)benzoic acid (40.0 g, 206.2 mmol) in DCM (300 mL) is added a few drops of DMF. Then oxalyl chloride (31.4 g, 247.4 mmol) is added dropwise at 0 °C. The resulting mixture is stirred for 2 hours at rt. The mixture is concentrated under reduced pressure to afford the crude methyl 2-(4-(chlorocarbonyl)phenyl)acetate. In other bottle, the solution of malononitrile (13.61 g, 206.2 mmol) in THF (100 mL) is added dropwise into a stirred suspension of NaH (16.5 g, 412.4 mmol, 60% in oil) in THF (100 mL) at 0-10 °C under N₂. The hydride mixture is then stirred for 20 minutes at rt. Then the crude methyl 2-(4-(chlorocarbonyl)phenyl)acetate in THF (200 mL) is added to the reaction mixture dropwise at 0-10 °C. The reaction is stirred for 1 hour at rt. Dimethyl sulfate (31.2 g, 247.4 mmol) is added to the reaction. The mixture is refluxed overnight at 80 °C under N₂. To the mixture is added water (300 mL) and the organics are extracted by EtOAc (3 x 300 mL). The combined organic layers are washed with sat. aq. NaCl, dried over Na₂SO₄, filtered and concentrated under reduced pressure. The resulting residue is purified by silica gel column chromatography (PE/EtOAc: 4/1-1/1) to give the title compound (42.0 g, 88% yield) as a yellow solid. ¹H NMR 400 MHz, (CDCl₃) δ 7.51 - 7.40 (m, 4H), 3.96 (s, 3H), 3.75 (s, 3H), 3.74 (s, 2H).

### Preparation 20

### Methyl 2-[4-[5-amino-4-cyano-1-[(1S)-2,2,2-trifluoro-1-methyl-ethyl]pyrazol-3-yl]phenyl]acetate

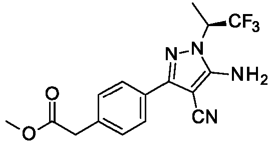

To a stirred solution of methyl 2-[4-(2,2-dicyano-1-methoxy-vinyl)phenyl]acetate (300 mg, 1.17 mmol) in THF (5 mL) is added [(1S)-2,2,2-trifluoro-1-methyl-ethyl]hydrazine hydrochloride (231.2 mg, 1.40 mmol) and TEA (236.9 mg, 2.34 mmol) at rt under N₂. The resulting mixture is stirred for 2 hours at 50 °C under N₂. The mixture is allowed to cool down to rt and is concentrated under reduced pressure. The residue is purified by silica gel column chromatography, eluting with PE / EtOAc (4:1-1:1), to give the title compound (210 mg, 51% yield) as a white solid. ES/MS *m*/*z* = 353.1 [M+H]⁺.

### Preparation 21

### 2-[4-[5-Amino-4-carbamoyl-1-[(1S)-2,2,2-trifluoro-1-methyl-ethyl]pyrazol-3-yl]phenyl]acetic acid

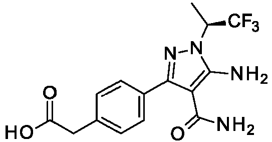

To a stirred solution of methyl 2-[4-[5-amino-4-cyano-1-[(1S)-2,2,2-trifluoro-1-methyl-ethyl]pyrazol-3-yl]phenyl]acetate (100 mg, 0.284 mmol) in EtOH (3 mL) and DMSO (0.5 mL) is added NaOH (34.1 mg, 0.85 mmol) and H₂O₂ (0.5 mL, 30% in H₂O) at rt under N₂. The resulting mixture is stirred for 2 hours at 50 °C under N₂. The mixture is allowed to cool down to rt and then is acidified to pH 5 with aq. HCl (1N). The resulting mixture is extracted with EtOAc (3 x 10 mL). The combined organic layers are washed with sat. aq. NaCl (2 x 10 mL) and dried over anhydrous Na₂SO₄. After filtration, the filtrate is concentrated under reduced pressure. The crude product is purified by Prep-HPLC with the following conditions (Column: XSelect CSH Prep C18 OBD^{™} Column, 19*150 mm, 5µm; Mobile Phase A: Water (0.05% FA), Mobile Phase B: ACN; Flow rate: 25 mL/min; Gradient: 15% B to 44% B in 8 min, 44% B; Wavelength: 254/220 nm. The fraction containing product is lyophilized to give the title compound (18.4 mg, 18% yield) as a white solid. ES/MS *m*/*z* = 357.05 [M+H]⁺. ¹H NMR 400 MHz, (DMSO-d₆) δ 7.43 (d, 2H), 7.35 (d, 2H), 6.66 (brs, 3H), 5.34 - 5.23 (m, 2H), 3.62 (s, 2H), 1.61 (d, 3H).

### Preparation 22

### 2-[4-[5-Amino-4-cyano-1-(2,2,2-trifluoro-1-methyl-ethyl)pyrazol-3-yl]phenyl]acetic acid

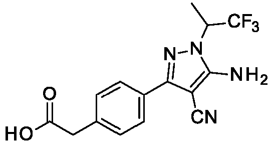

A solution of methyl 2-[4-[5-amino-4-cyano-1-(2,2,2-trifluoro-1-methyl-ethyl)py-razol-3-yl]phenyl]acetate (3.20 g, 9.08 mmol) and LiOH (0.65 g, 27.3 mmol) in MeOH/H₂O (4:1, 25 mL) is stirred for 2 hours at rt. The reaction is concentrated under reduced pressure to remove the solvent and then EtOAc (10 mL) is added. The filter cake is dissolved in water (50 mL) and is acidified to pH 6 by aq. HCl (4M). The resulting mixture is extracted with EtOAc (3 x 100 mL). The combined organic layers are washed with sat. aq. NaCl (2 x 50 mL) and dried over anhydrous Na₂SO₄. After filtration, the filtrate is concentrated under reduced pressure to give the crude compound (3 g, 97%) as a brown solid. ES/MS *m*/*z* = 339.2 [M+H]⁺.

### Preparation 23

### Benzyl N-[[4-[5-amino-4-cyano-1-(2,2,2-trifluoro-1-methyl-ethyl)pyrazol-3-yl]phenyl]methyl]carbamate

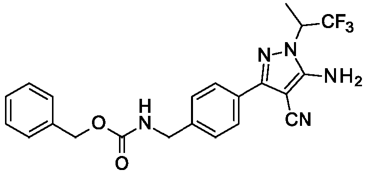

To a stirred solution of 2-[4-[5-amino-4-cyano-1-(2,2,2-trifluoro-1-methylethyl)pyrazol-3-yl]phenyl]acetic acid (1.00 g, 2.956 mmol, 1.00 equiv) and benzyl alcohol (383.60 mg, 3.547 mmol, 1.20 equiv) in toluene (20.00 mL) is added TEA (598.2 mg, 5.91 mmol) and DPPA (1.22 g, 4.43 mmol) dropwise at rt under N₂. The resulting mixture is stirred overnight at 110 °C under N₂. The mixture is allowed to cool down to rt and is concentrated under reduced pressure. The resulting residue is purified by silica gel column chromatography, eluting with PE/EtOAc (2:1-1:1) to give the title compound (300 mg, 23% yield) as a yellow solid. ES/MS *m*/*z* = 444.1 [M+H]⁺. ¹H NMR 400 MHz, (DMSO-d₆) δ 7.90 - 7.86 (m, 1H), 7.79 - 7.69 (m, 2H), 7.38 - 7.32 (m, 6H), 7.10 (s, 2H), 5.35 - 5.06 (m, 1H), 5.06 (s, 2H), 4.31 - 4.24 (m, 2H), 1.66 (d, 3H).

## Claims

1. A process for the preparation of (S)-5-amino-3-(4-((5-fluoro-2-methoxyben-zamido)methyl)phenyl)-1-(1,1,1-trifluoropropane-2-yl)-1H-pyrazole-4-carboxamide (I) comprising the steps of:
viii) coupling the compound of Formula (III): 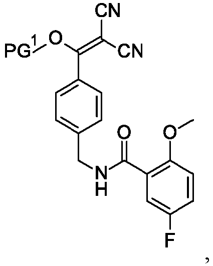 wherein PG¹ is -CH₃, -CH₂CH₃, -C(CH₃)₃, -CH₂CH=CH₂, methoxymethyl, tetrahydropyran, benzyl, trimethylsilyl, *tert*-butyl dimethylsilyl, di*-tert*-butylisobutylsilyl, di-*tert*-butyl[pyren-1-ylmethoxy]silyl, *tert*-butyl diphenylsilyl, acetyl, or benzoyl; and [(1S)-2,2,2-trifluoro-1-methyl-ethyl]hydrazine (8) or salt thereof to give N-[[4-[5-amino-4-cyano-1-[(1S)-2,2,2-trifluoro-1-methyl-ethyl]pyrazol-3-yl]phenyl]methyl]-5-fluoro-2-methoxybenzamide (10) or a salt thereof; and
ix) synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methylethyl]pyrazol-3-yl]phenyl]methyl]-5-fluoro-2-methoxy-benzamide (10) or a salt thereof.

2. The process according to claim 1, further comprising crystallizing (S)-5-amino-3-(4-((5-fluoro-2-methoxybenzamido)methyl)phenyl)-1-(1,1,1-trifluoropropane-2-yl)-1H-pyrazole-4-carboxamide (I) to provide a (S)-5-amino-3-(4-((5-fluoro-2-methoxybenzamido)methyl)phenyl)-1-(1,1,1-trifluoropropane-2-yl)-1H-pyrazole-4-carboxamide (I) in crystalline form.

3. The process according to claim 1 or 2, wherein PG¹ is -CH₃.

4. The process according to claim 1 or 2, wherein synthesizing (S)-5-amino-3-(4-((5-fluoro-2-methoxybenzamido)methyl)phenyl)-1-(1,1,1-trifluoropropane-2-yl)-1H-pyrazole-4-carboxamide (I) from N-[[4-[5-amino-4-cyano-1-[(1S)-2,2,2-trifluoro-1-methyl-ethyl]pyrazol-3-yl]phe-nyl]methyl]-5-fluoro-2-methoxy-benzamide (10) or a salt thereof is via a hydrolysis.

5. The process according to claim 4, wherein the hydrolysis is carried out under acidic, basic, oxidative, or metal-catalyzed/stoichiometric conditions.

6. The process according to claim 5, wherein the hydrolysis is carried out under acidic conditions.

7. The process according to claim 6, wherein the hydrolysis employs methanesulfonic acid, trifluoroacetic acid, hydrochloric acid, polyphosphoric acid, or sulfuric acid.

8. The process according to claim 7, wherein the hydrolysis employs methanesulfonic acid.

9. The process according to claim 5, wherein the hydrolysis is carried out under basic, oxidative, or metal-catalyzed/stoichiometric conditions.

10. The process according to claim 9, wherein the hydrolysis employs potassium *tert*butoxide, sodium hydroxide, peroxide, ruthenium hydroxide, manganese dioxide, copper (II) acetate, Parkin's catalyst or MnO₂/SiO₂.

11. The process according to any one of claims 1-10, wherein prior to the coupling of the compound of Formula (III): 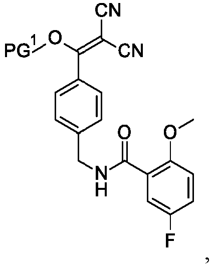 and [(1S)-2,2,2-trifluoro-1-methyl-ethyl]hydrazine (8) or a salt thereof, the process further comprises the step of:
reacting N-[[4-(2,2-dicyano-1-hydroxy-vinyl)phenyl]methyl]-5-fluoro-2-methoxy-benzamide (II) with an alkylating agent to give the compound of Formula (III):

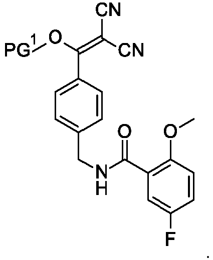

12. The process according to claim 11, wherein reacting N-[[4-(2,2-dicyano-1-hydroxy-vinyl)phenyl]methyl]-5-fluoro-2-methoxy-benzamide (II) with an alkylating agent gives the compound of Formula (IIIA):

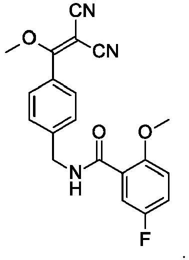

13. The process according to claim 12, wherein the reaction employs trimethyl orthoformate, methyl triflate, trimethylammonium tetrafluoroborate, or *N,N'*-diisopropyl-*O*-methylisourea.

14. The process according to any one of claims 1-11, wherein prior to the coupling of the compound of Formula (III) and [(1S)-2,2,2-trifluoro-1-methyl-ethyl]hydrazine (8), the process further comprises the step of:
converting [(1S)-2,2,2-trifluoro-1-methyl-ethyl]hydrazine hydrochloride (7) to [(1S)-2,2,2-trifluoro-1-methyl-ethyl]hydrazine (8).

15. The process according to claim 14, wherein prior to converting [(1S)-2,2,2-trifluoro-1-methyl-ethyl]hydrazine hydrochloride (7) to [(1S)-2,2,2-trifluoro-1-methyl-ethyl]hydrazine (8), the process further comprises the step of:
reacting N'-[(1S)-2,2,2-trifluoro-1-methyl-ethyl]benzohydrazide (6) or a salt thereof to give [(1S)-2,2,2-trifluoro-1-methyl-ethyl]hydrazine hydrochloride (7).

16. A compound which is N-[[4-(2,2-dicyano-1-hydroxy-vinyl)phenyl]methyl]-5-fluoro-2-methoxy-benzamide:

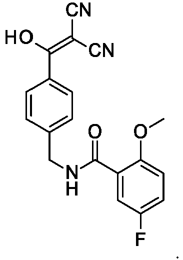

17. A compound: 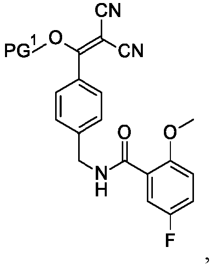 wherein PG¹ is -CH₃, -CH₂CH₃, -C(CH₃)₃, -CH₂CH=CH₂, methoxymethyl, tetrahydropyran, benzyl, trimethylsilyl, *tert*-butyl dimethylsilyl, di-*tert*-butylisobutylsilyl, di-tert-butyl[pyren-1-ylmethoxy]silyl, *tert*-butyl diphenylsilyl, acetyl, or benzoyl.

18. The compound according to claim 17 which is:

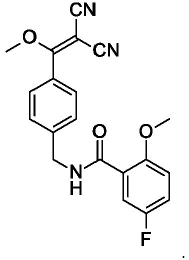

## Patentansprüche

1. Prozess für die Herstellung von (S)-5-Amino-3-(4-((5-fluor-2-methoxybenzamido)methyl)phenyl)-1-(1,1,1-trifluorpropan-2-yl)-1H-pyrazol-4-carboxamid (I), umfassend die Schritte:
viii) Koppeln der Verbindung der Formel (III): 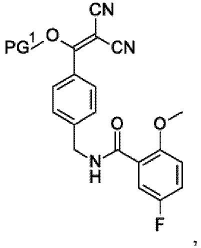 wobei PG¹ -CH₃, -CH₂CH₃, -C(CH₃)₃, -CH₂CH=CH₂, Methoxymethyl, Tetrahydropyran, Benzyl, Trimethylsilyl, *tert*-Butyldimethylsilyl, Di-*tert-*butylisobutylsilyl, Di-*tert*-butyl[pyren-1-ylmethoxy]silyl, *tert*-Butyldiphenylsilyl, Acetyl oder Benzoyl ist; und [(1S)-2,2,2-Trifluor-1-methyl-ethyl]hydra-zin (8) oder ein Salz davon, um N-[[4-[5-Amino-4-cyano-1-[(1S)-2,2,2-trifluor-1-methyl-ethyl]pyrazol-3-yl]phenyl]methyl]-5-fluor-2-methoxy-benzamid (10) oder ein Salz davon zu ergeben; und
ix) Synthetisieren von (S)-5-Amino-3-(4-((5-fluor-2-methoxybenzamido)methyl)phenyl)-1-(1,1,1-trifluorpropan-2-yl)-1H-pyrazol-4-carboxamid (I) aus N-[[4-[5-Amino-4-cyano-1-[(1S)-2,2,2-trifluor-1-methyl-ethyl]pyrazol-3-yl]phenyl]methyl]-5-fluor-2-methoxy-benzamid (10) oder einem Salz davon

2. Prozess nach Anspruch 1, ferner umfassend ein Kristallisieren von (S)-5-Amino-3-(4-((5-fluor-2-methoxybenzamido)methyl)phenyl)-1-(1,1,1-trifluorpropan-2-yl)-1H-pyrazol-4-carboxamid (I), um ein (S)-5-Amino-3-(4-((5-fluor-2-methoxybenzamido)methyl)phenyl)-1-(1,1,1-trifluorpropan-2-yl)-1H-pyrazol-4-carboxamid (I) in kristalliner Form bereitzustellen.

3. Prozess nach Anspruch 1 oder 2, wobei PG¹ -CH₃.ist.

4. Prozess nach Anspruch 1 oder 2, wobei das Synthetisieren von (S)-5-Amino-3-(4-((5-fluor-2-methoxybenzamido)methyl)phenyl)-1-(1,1,1-trifluorpropan-2-yl)-1H-pyrazol-4-carboxamid (I) aus N-[[4-[5-Amino-4-cyano-1-[(1S)-2,2,2-trifluor-1-methyl-ethyl]pyrazol-3-yl]phe-nyl]methyl]-5-fluor-2-methoxy-benzamid (10) oder einem Salz davon über eine Hydrolyse erfolgt.

5. Prozess nach Anspruch 4, wobei die Hydrolyse unter sauren, basischen, oxidativen oder metallkatalysierten/stöchiometrischen Bedingungen ausgeführt wird.

6. Prozess nach Anspruch 5, wobei die Hydrolyse unter sauren Bedingungen ausgeführt wird.

7. Prozess nach Anspruch 6, wobei die Hydrolyse Methansulfonsäure, Trifluoressigsäure, Salzsäure, Polyphosphorsäure oder Schwefelsäure anwendet.

8. Prozess nach Anspruch 7, wobei die Hydrolyse Methansulfonsäure anwendet.

9. Prozess nach Anspruch 5, wobei die Hydrolyse unter basischen, oxidativen oder metallkatalysierten/stöchiometrischen Bedingungen ausgeführt wird.

10. Prozess nach Anspruch 9, wobei die Hydrolyse Kalium-*tert-*butoxid, Natriumhydroxid, Peroxid, Rutheniumhydroxid, Mangandioxid, Kupfer(II)-acetat, Parkin-Katalysator oder MnO₂/SiO₂ anwendet.

11. Prozess nach einem der Ansprüche 1 bis 10, wobei vor dem Koppeln der Verbindung der Formel (III): 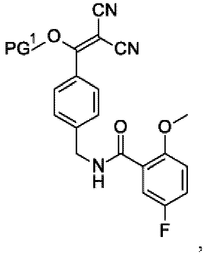 und [(1S)-2,2,2-Trifluor-1-methyl-ethyl]hydrazin (8) oder ein Salz davon, der Prozess ferner den Schritt umfasst:
Umsetzen von N-[[4-(2,2-Dicyano-1-hydroxy-vinyl)phenyl]methyl]-5-fluor-2-methoxy-benzamid (II) mit einem Alkylierungsmittel, um die Verbindung der Formel (III) zu ergeben:

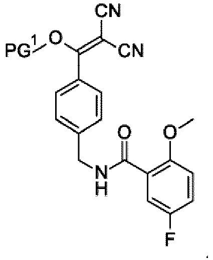

12. Prozess nach Anspruch 11, wobei das Umsetzen von N-[[4-(2,2-Dicyano-1-hydroxy-vinyl)phenyl]methyl]-5-fluor-2-methoxy-benzamid (II) mit einem Alkylierungsmittel die Verbindung der Formel (IIIA) ergibt:

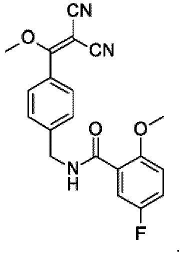

13. Prozess nach Anspruch 12, wobei die Reaktion Trimethylorthoformiat, Methyltriflat, Trimethylammoniumtetrafluoroborat oder *N,N*'-Diisopropyl-*O-*methylisoharnstoff anwendet.

14. Prozess nach einem der Ansprüche 1 bis 11, wobei, vor dem Koppeln der Verbindung der Formel (III) und [(1S)-2,2,2-Trifluor-1-methyl-ethyl]hydrazin (8), der Prozess ferner den Schritt umfasst:
Umwandeln von [(1S)-2,2,2-Trifluor-1-methyl-ethyl]hydrazinhydrochlorid (7) in [(1S)-2,2,2-Trifluor-1-methyl-ethyl]hydrazin (8).

15. Prozess nach Anspruch 14, wobei vor dem Umwandeln von [(1S)-2,2,2-Trifluor-1-methyl-ethyl]hydrazinhydrochlorid (7) in [(1S)-2,2,2-Trifluor-1-methyl-ethyl]hydrazin (8) der Prozess ferner den Schritt umfasst:
Umsetzen von N'-[(1S)-2,2,2-Trifluor-1-methyl-ethyl]benzohydrazid (6) oder einem Salz davon, um [(1S)-2,2,2-Trifluor-1-methyl-ethyl]hydrazinhydrochlorid (7) zu ergeben.

16. Verbindung, die N-[[4-(2,2-dicyano-1-hydroxy-vinyl)phenyl]methyl]-5-fluor-2-methoxy-benzamid ist:

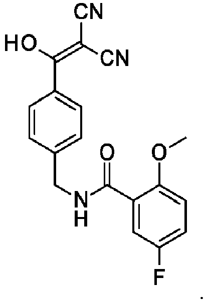

17. Verbindung: 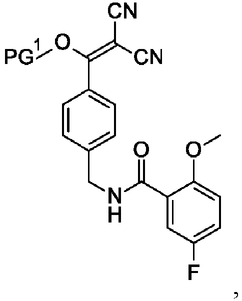 wobei PG¹ -CH₃, -CH₂CH₃, -C(CH₃)₃, -CH₂CH=CH₂, Methoxymethyl, Tetrahydropyran, Benzyl, Trimethylsilyl, *tert*-Butyldimethylsilyl, Di-*tert-*butylisobutylsilyl, Di-*tert*-butyl[pyren-1-ylmethoxy]silyl, *tert*-Butyldiphenylsilyl, Acetyl oder Benzoyl ist.

18. Verbindung nach Anspruch 17, die ist:

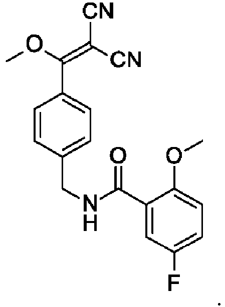

## Revendications

1. Procédé de préparation de (S)-5-amino-3-(4-((5-fluoro-2-méthoxyben-zamido)méthyl)phényl)-1-(1,1,1-trifluoropropane-2-yl)-1H-pyrazole-4-carboxamide (I) comprenant les étapes consistant à :
viii) coupler le composé de Formule (III) : 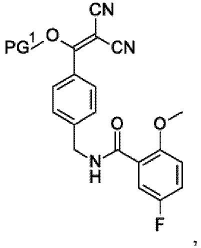 dans lequel PG¹ est -CH₃, -CH₂CH₃, -C(CH₃)₃, -CH₂CH=CH₂, méthoxyméthyle, tétrahydropyrane, benzyle, triméthylsilyle, tert-butyldiméthylsilyle, di-*tert*-butylisobutylsilyle, di-*tert*-butyl[pyrén-1-ylméthoxy]silyle, *tert*-butyldiphénylsilyle, acétyle, ou benzoyle ; et de [(1S)-2,2,2-trifluoro-1-méthyl-éthyl]hydrazine (8) ou d'un sel de celle-ci, pour donner le N-[[4-[5-amino-4-cyano-1-[(1S)-2,2,2-tri-fluoro-1-méthyl-éthyl]pyrazol-3-yl]phényl]méthyl]-5-fluoro-2-méthoxy-benzamide (10) ou un sel de celui-ci ; et
ix) synthétiser (S)-5-amino-3-(4-((5-fluoro-2-méthoxybenzamido)méthyl)phényl)-1-(1,1,1-trifluoropropane-2-yl)-1H-pyrazole-4-carboxamide (I) à partir de N-[[4-[5-amino-4-cyano-1-[(1S)-2,2,2-trifluoro-1-méthyl-éthyl]pyrazol-3-yl]phényl]méthyl]-5-fluoro-2-méthoxy-benzamide (10) ou d'un sel de celui-ci

2. Procédé selon la revendication 1, comprenant en outre la cristallisation de (S)-5-amino-3-(4-((5-fluoro-2-méthoxybenzamido)méthyl)phényl)-1-(1,1,1-trifluoropropane-2-yl)-1H-pyrazole-4-carboxamide (I) pour fournir un (S)-5-amino-3-(4-((5-fluoro-2-méthoxybenzamido)méthyl)phényl)-1-(1,1,1-trifluoropropane-2-yl)-1H-pyrazole-4-carboxamide (I) sous forme cristalline.

3. Processus selon la revendication 1 ou 2, dans lequel PG¹ est -CH₃.

4. Processus selon la revendication 1 ou 2, dans lequel la synthèse de (S)-5-amino-3-(4-((5-fluoro-2-méthoxybenzamido)méthyl)phényl)-1-(1,1,1-trifluoropropane-2-yl)-1H-pyrazole-4-carboxamide (I) à partir du N-[[4-[5-amino-4-cyano-1-[(1S)-2,2,2-trifluoro-1-méthyl-éthyl]pyrazol-3-yl]phényle]méthyl]-5-fluoro-2-méthoxy-benzamide (10) ou d'un sel de celui-ci est réalisée par une hydrolyse.

5. Processus selon la revendication 4, dans lequel l'hydrolyse est réalisée dans des conditions acides, basiques, oxydantes ou catalysées par un métal/stœchiométriques.

6. Processus selon la revendication 5, dans lequel l'hydrolyse est réalisée dans des conditions acides.

7. Processus selon la revendication 6, dans lequel l'hydrolyse emploie de l'acide méthanesulfonique, de l'acide trifluoroacétique, de l'acide chlorhydrique, de l'acide polyphosphorique ou de l'acide sulfurique.

8. Processus selon la revendication 7, dans lequel l'hydrolyse emploie de l'acide méthanesulfonique.

9. Processus selon la revendication 5, dans lequel l'hydrolyse est réalisée dans des conditions basiques, oxydantes ou catalysées par un métal/stœchiométriques.

10. Processus selon la revendication 9, dans lequel l'hydrolyse emploie du *tert-*butoxyde de potassium, de l'hydroxyde de sodium, du peroxyde, de l'hydroxyde de ruthénium, du dioxyde de manganèse, de l'acétate de cuivre (II), le catalyseur de Parkin ou MnO₂/SiO₂.

11. Processus selon l'une quelconque des revendications 1 à 10, dans lequel avant le couplage du composé de Formule (III) : 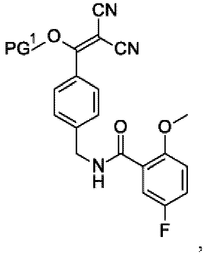 et de [(1S)-2,2,2-trifluoro-1-méthyl-éthyl]hydrazine (8) ou d'un sel de celle-ci, le processus comprend en outre l'étape consistant à :
faire réagir N-[[4-(2,2-dicyano-1-hydroxy-vinyl)phényl]méthyl]-5-fluoro-2-méthoxy-benzamide (II) avec un agent alkylant pour donner le composé de formule (III) :

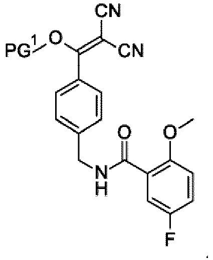

12. Processus selon la revendication 11, dans lequel la réaction de N-[[4-(2,2-dicyano-1-hydroxy-vinyl)phényl]méthyl]-5-fluoro-2-méthoxy-benzamide (II) avec un agent alkylant donne le composé de Formule (IIIA) :

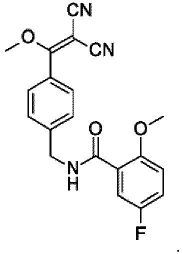

13. Processus selon la revendication 12, dans lequel la réaction emploie de l'orthoformiate de triméthyle, du triflate de méthyle, du tétrafluoroborate de triméthylammonium ou de la *N,N*'-diisopropyl-*O*-méthylisourée.

14. Processus selon l'une quelconque des revendications 1 à 11, dans lequel, avant le couplage du composé de Formule (III) et de [(1S)-2,2,2-trifluoro-1-méthyl-éthyl]hydrazine (8), le procédé comprend en outre l'étape consistant à :
convertir le chlorhydrate de [(1S)-2,2,2-trifluoro-1-méthyl-éthyl]hydrazine (7) en [(1S)-2,2,2-trifluoro-1-méthyl-éthyl]hydrazine (8).

15. Processus selon la revendication 14, dans lequel avant de convertir le chlorhydrate de [(1S)-2,2,2-tri-fluoro-1-méthyl-éthyl]hydrazine (7) en [(1S)-2,2,2-trifluoro-1-méthyl-éthyl]hydrazine (8), le processus comprend en outre l'étape consistant à :
faire réagir du N'-[(1S)-2,2,2-trifluoro-1-méthyl-éthyl]benzohydrazide (6) ou d'un sel de celui-ci pour donner le chlorhydrate de [(1S)-2,2,2-trifluoro-1-méthyl-éthyl]hydrazine (7).

16. Composé qui est le N-[[4-(2,2-dicyano-1-hydroxy-vinyl)phényl]méthyl]-5-fluoro-2-méthoxy-benzamide :

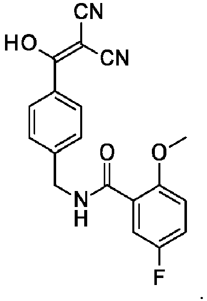

17. Composé : 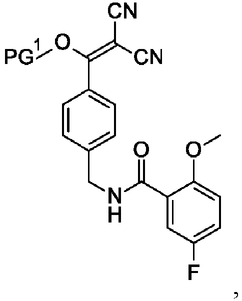 dans lequel PG¹ est -CH₃, -CH₂CH₃, -C(CH₃)₃, -CH₂CH=CH₂, méthoxyméthyle, tétrahydropyrane, benzyle, triméthylsilyle, *tert-*butyldiméthylsilyle, di-*tert*-butylisobutylsilyle, di-*tert*-butyl[pyrén-1-ylméthoxy]silyle, *tert*-butyldiphénylsilyle, acétyle ou benzoyle.

18. Composé selon la revendication 17 qui est :
